(19) Europäisches Patentamt
European Patent Office
Office européen des brevets

(11) EP 0 946 487 B1

(12) EUROPÄISCHE PATENTSCHRIFT

(45) Veröffentlichungstag und Bekanntmachung des Hinweises auf die Patenterteilung:
**07.08.2002 Patentblatt 2002/32**

(21) Anmeldenummer: **97953796.6**

(22) Anmeldetag: **12.12.1997**

(51) Int Cl.[7]: **C07C 67/03**, B01J 31/22, B01J 31/16, C07F 7/22

(86) Internationale Anmeldenummer:
**PCT/EP97/06978**

(87) Internationale Veröffentlichungsnummer:
**WO 98/28256 (02.07.1998 Gazette 1998/26)**

(54) **AN FESTE TRÄGERMATERIALIEN FIXIERTE UMESTERUNGSKATALYSATOREN**

TRANSESTERIFICATION CATALYSTS FIXED TO SOLID SUPPORT MATERIALS

CATALYSEURS DE TRANSESTERIFICATION FIXES SUR DES MATERIAUX DE SUPPORT SOLIDES

(84) Benannte Vertragsstaaten:
**BE DE ES FR GB IT NL**

(30) Priorität: **20.12.1996 CH 315796**

(43) Veröffentlichungstag der Anmeldung:
**06.10.1999 Patentblatt 1999/40**

(60) Teilanmeldung:
**02001188.8 / 1 209 144**

(73) Patentinhaber: **Ciba Specialty Chemicals Holding Inc.
4057 Basel (CH)**

(72) Erfinder:
- **PUGIN, Benoît
CH-4142 Münchenstein (CH)**
- **DUBUIS, Benoît
CH-4132 Muttenz (CH)**
- **MÜLLER, Adrian
CH-4056 Basel (CH)**

(56) Entgegenhaltungen:

| | |
|---|---|
| **EP-A- 0 646 567** | **DE-A- 3 119 643** |
| **DE-C- 4 317 428** | **US-A- 5 561 205** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft immobilisierte Zinn-Schwefel-Katalysatoren, ihre Herstellung und ihre Verwendung bei Umesterungsreaktionen von Carbonsäureestern.

Umesterungsreaktionen von Carbonsäureestern lassen sich durch das folgende Reaktionsschema allgemein beschreiben:

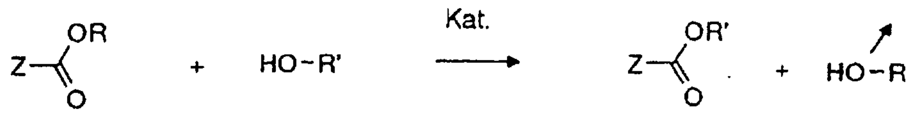


wobei R üblicherweise ein $C_1$-$C_4$-Alkylrest ist und R' und Z einen organischen Rest bedeuten. Die Reaktion ist eine Gleichgewichtsreaktion. In der Regel wird der freiwerdende (tiefersiedende) Alkohol während der Reaktion abdestilliert. Es sind eine ganze Reihe von verschiedenen Katalysatoren für diese Reaktion bekannt (Junzo Otera, Chem. Rev., 93 (1993) 1449-1470.), z.B. Säuren, Basen, Amine, Metall-Alkoxide und unter anderem auch Organozinn-Verbindungen. Viele dieser Umesterungsreaktionen werden bei Temperaturen im Bereich von 80°C bis über 200°C durchgeführt.

Es ist ökonomisch von Vorteil, wenn die Reaktionen ohne Lösungsmittel in der Schmelze durchgeführt werden können. Ein weiterer Vorteil ist, wenn das Produkt nach der Reaktion nicht durch Destillation oder Extraktion gereinigt werden muss. Daher sollte ein Katalysator bei hohen Temperaturen verwendet werden können (Reaktion in der Schmelze) und die katalysierte Reaktion darf nicht zu verfärbten Produkten führen.

Organozinn-Verbindungen sind bekannte und sehr schonende Katalysatoren für Umesterungen von Carbonsäureestern mit Alkoholen. Ein Problem mit herkömmlichen Zinnkatalysatoren besteht darin, eine oekonomische Abtrennung des Zinns aus dem Reaktionsprodukt zu erreichen. Eine Lösungsmöglichkeit ist die Verwendung von immobilisierten, abtrennbaren Zinnkatalysatoren.

Aus US 5436357 sind beispielsweise auf Polystyrol gebundene Katalysatoren vom Typ:

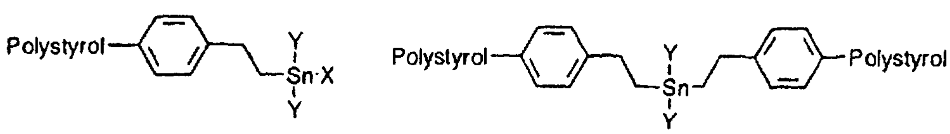

Y und X= OMe
Y= OMe, X= Cl, Br

bekannt. Diese Verbindungen werden als Katalysatoren für Umesterungen in einem Temperaturbereich von 50-150°C vorgeschlagen. Sie können abdekantiert oder filtriert und mehrmals wieder eingesetzt werden. In den meisten Fällen ist Wiedereinsetzen mit einem Verlust an Aktivität verbunden. Über das "Sn-Leaching" werden keine Angaben gemacht, beispielsweise als Restgehalt an Zinn im Produkt.

Weiterhin sind von H. Schuhmann und B. Pachaly, J. Organomet. Chem., 233 (1982) 281-289 und H. Schuhmann und B. Pachaly, Angew. Chem., 93 (1981) 1092-93 die Herstellung folgender Verbindungen beschrieben worden, welche stöchiometrisch als Hydride zur Reduktion von Alkylhalogeniden eingesetzt wurden.

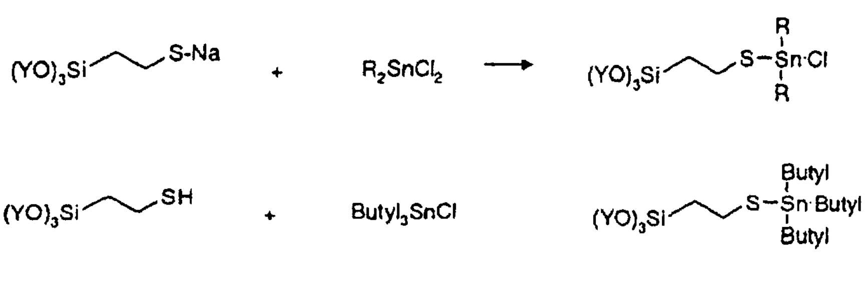

R= Butyl, Phenyl
Y= Me, Et

Diese Verbindungen werden auf Silicagel (oder Aluminiumoxid) immobilisiert und durch Reduktion zum entsprechenden Hydrid-Reagenz überführt:

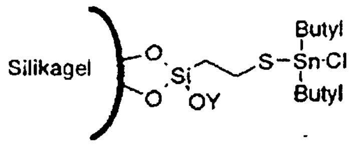

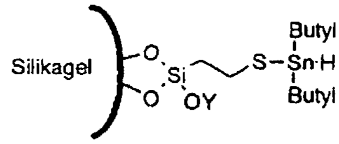

Im wesentlichen gleiche Verbindungen wie in den vorstehend angegebenen Publikationen werden in der DE 31 19 643 beschrieben.

Aus US 5, 561, 205 ist ein Zinn (IV)-Katalysator zur Umesterung von Carbonsäureestem bekannt, wobei die Zinn (IV)-Verbindung an einen organischen polymeren Träger gebunden ist.

Gegenstand der vorliegenden Erfindung ist ein Verfahren zur Umesterung von Carbonsäureestern, dadurch gekennzeichnet, dass man als Katalysator eine an einen anorganischen Träger gebundene Zinn(IV)-Verbindung, enthaltend einen Rest der Formel

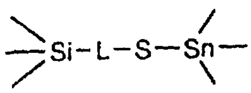

(I),

verwendet, worin L ein mindestens zweiwertiger Rest ist und mindestens eine der freien Valenzen des Si in Formel (I) an den anorganischen Täger gebunden ist.

Diese immobilisierten Zinn-Schwefel-Katalysatoren können in einem grossen TemperaturBereich verwendet werden, und nach der Reaktion einfach und vollständig durch Fest/Flüssig-Trennoperationen, wie beispielsweise Filtration, Zentrifugieren und Dekantieren, vom Reaktionsprodukt abgetrennt werden. Die abfiltrierten Katalysatoren sind weiterhin katalytisch aktiv und können mehrmals wiederverwendet werden.

Das erfindungsgemässe Verfahren ist besonders zur Herstellung von Verbindungen der allgemeinen Formel

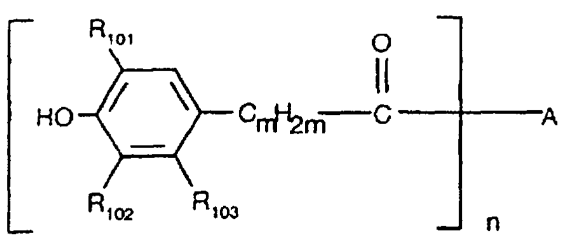

(X)

geeignet,

wobei

$R_{101}$ und $R_{102}$ gleich oder verschieden sind und H, Alkyl mit 1 bis 18 C-Atomen, Phenyl, $C_1$-$C_4$-alkylsubstituiertes Phenyl, Phenylalkyl mit 7 bis 9 C-Atomen, $C_5$-$C_{12}$-Cycloalkyl, $C_1$-$C_4$alkylsubstituiertes $C_5$-$C_{12}$-Cycloalkyl oder einen Rest

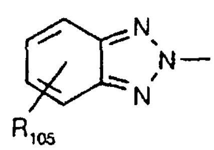

darstellen;

$R_{103}$ H oder $CH_3$ darstellt;

$R_{105}$ H, Cl, $-SO_3H$ oder $C_1$-$C_4$-Alkyl darstellt;
m eine Zahl 0, 1, 2 oder 3 ist; und
n eine Zahl 1, 2, 3 oder 4 bedeutet; wobei,
wenn n = 1 ist,
A $-OR_{104}$ ist; und
$R_{104}$ die Bedeutung von Alkyl mit 2 bis 45 C-Atomen, $C_2$-$C_{45}$-Alkyl mit einem oder mehreren Sauerstoff unterbrochen, Cycloalkyl mit 5 bis 12 C-Atomen, Alkenyl mit 2 bis 18 C-Atomen, $-CH_2CHOHCH_2OC(O)R_{109}$ oder $-CH_2CH_2-OR_{106}$ hat; und
$R_{109}$ H, Alkyl mit 1 bis 8 C-Atomen, Alkenyl mit 3 bis 5 C-Atomen oder Benzyl ist; wobei
$R_{106}$

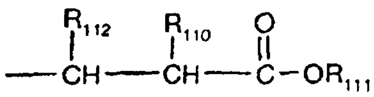 , 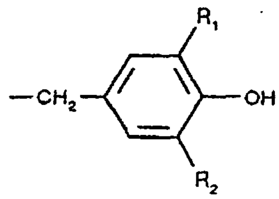

,H, Alkyl mit 1 bis 24 C-Atomen, Phenyl, Cycloalkyl mit 5 bis 12 C-Atomen oder

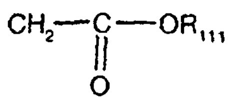

ist;
und
$R_{110}$ H oder $-CH_3$;
$R_{111}$ H oder Alkyl mit 1 bis 24 C-Atomen; und
$R_{112}$ H oder $-CH_3$ sind, mit der Massgabe, dass $R_{110}$ und $R_{112}$ nicht gleichzeitig $-CH_3$ sind; oder, wenn n = 2 ist,
A $-O-C_xH_{2x}-O-$ , $-O-(CH_2CH_2O)_aCH_2CH_2O-$ oder $-O-CH_2CH=CHCH_2-O-$ ist; wobei
a eine Zahl von 1 bis 30 ist; und
x eine Zahl von 2 bis 20 ist;
oder, wenn n = 3 ist,
A

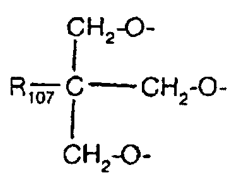

bedeutet; wobei
$R_{107}$ Alkyl mit 1 bis 24 C-Atomen oder Phenyl darstellt,
oder, wenn n = 4 ist,
A

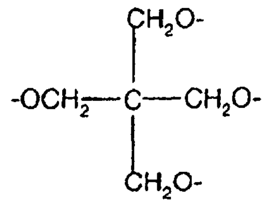

bedeutet.

Besonders bevorzugt ist das Verfahren zur Herstellung der Verbindungen der Formel (X), worin n die Zahl eins ist;
$R_{101}$ tert. Butyl oder

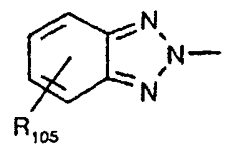

$R_{102}$ tert.-Butyl;
$R_{103}$ und $R_{105}$ H;
m die Zahl 2; A $OR_{104}$;
$R_{104}$ $(CH_2)_{17}CH_3$ ,$C_8H_{17}$ (Isomerenmischung), $C_2$-$C_{45}$-Alkyl mit einem oder mehreren Sauerstoff unterbrochen, $-CH_2CHOHCH_2OC(O)R_{109}$; und
$R_{109}$ Alkenyl mit 3 bis 5 C-Atomen ist;
oder n die Zahl 2 ist;
$R_{101}$ tert. Butyl oder

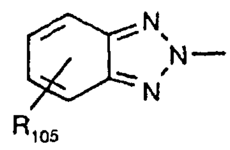

$R_{102}$ $CH_3$;
$R_{103}$ H;
m die Zahl 2;
A , $-O-(CH_2CH_2O)_aCH_2CH_2O-$ ; und
a eine Zahl von 1 bis 30 ist;
oder n die Zahl 3 ist;
$R_{101}$ und $R_{102}$ tert.-Butyl;
$R_{103}$ H;
m die Zahl 2; und
A $-O-CH_2-CH(O-)-CH_2-O-$ ist;
oder n die Zahl 4 ist;
$R_{101}$ und $R_{102}$ tert.-Butyl;
$R_{103}$ H;
m die Zahl 2; und
A $C(CH_2-O-)_4$ ist.

Das erfindungsgemässe Verfahren ist ebenfalls zur Herstellung von Verbindungen der allgemeinen Formel

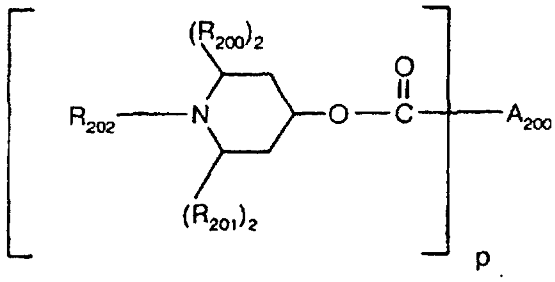

(XX)

geeignet,
worin
$R_{200}$ und $R_{201}$ unabhängig voneinander $C_1$-$C_4$-Alkyl;
$R_{202}$ H, $C_1$-$C_{18}$-Alkyl, O oder $C_1$-$C_{18}$-Alkoxy;
$A_{200}$ wenn p=1 ist, $C_1$-$C_{12}$-Alkyl, $C_1$-$C_{12}$-Alkyl substituiert mit -C(O)OH oder $C_2$-$C_5$-Alkenyl; oder, wenn p=2 ist, $C_1$-$C_{12}$-Alkylen; und
p die Zahl 1 oder 2 bedeutet.

Das erfindungsgemässe Verfahren ist ebenfalls zur Herstellung von Verbindungen geeignet, welche durch die Umsetzung einer Verbindung der Formel

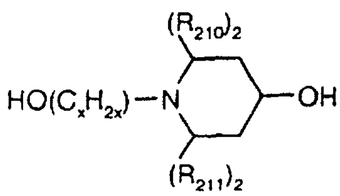

(XXX)

worin
$R_{210}$ und $R_{211}$ unabhängig voneinander $C_1$-$C_4$-Alkyl; und
x eine Zahl 1 bis 12 ist;
mit einer Verbindung der Formel

$$R_{212}OC(O)\text{-}A_{210}\text{-}(O)COR_{213} \qquad (XXXI)$$

worin
$R_{212}$ und $R_{213}$ unabhängig voneinander $C_1$-$C_4$-Alkyl; und
$A_{210}$ $C_1$-$C_{12}$-Alkylen ist;
erhältlich sind.

Bei den durch Reaktion der Verbindungen (XXX) mit (XXXI) erhältlichen Verbindungen handelt es sich im allgemeinen um oligomere oder polymere Verbindungen beziehungsweise Gemische solcher Verbindungen. Durch die stöchiometrischen Verhältnisse und Reaktionsparameter lässt sich die Entstehung der Verbindungen beziehungsweise Verbindungsgemische beeinflussen.

Beispiele zu den Substituentenbedeutungen der Formeln (X), (XX), (XXX) und (XXXI) sind nachfolgend angeführt.

Alkyl mit bis zu 45 C-Atomen kann eine geradkettige oder verzweigte Alkylgruppe sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl, Eicosyl, Henicosyl, Docosyl, Triacontyl und weitere. Bevorzugt sind Alkyl mit 1-12 C-Atomen, und besonders bevorzugt Alkyl mit 1-8 C-Atomen. Soweit i-$C_8H_{17}$ genannt wird, wird darunter auch eine Mischung von Isomeren verstanden werden.

Ganz besonders bevorzugt für $R_{101}$ und $R_{102}$ wird die t-Butylgruppe. Weiters bevorzugt für $R_{101}$ ist -$CH_3$ und für $R_{102}$ t-Butyl oder für $R_{101}$ Isopropyl und für $R_{102}$ t-Butyl. Für $R_{103}$ ist -H bevorzugt. Bevorzugt für $R_{111}$, $R_{107}$ oder $R_{106}$ sind Alkylgruppen mit 1 bis 18 C-Atomen.

Bedeutet ein Substituent Cycloalkyl mit 5 bis 12 C-Atomen, so zählen beispielsweise dazu Cyclopentyl, Cyclohexyl, Cycloheptyl, Cyclooctyl, Cyclodecyl und Cyclododecyl. Bevorzugt wird Cyclohexyl.

Beispiele für $C_1$-$C_4$-alkylsubstituiertes Cycloalkyl mit 5 bis 12 C-Atomen sind 2- oder 4-Methylcyclohexyl, Dimethylcyclohexyl, Trimethylcyclohexyl oder t-Butylcyclohexyl.

Bedeutet ein Substituent $C_1$-$C_4$-alkylsubstituiertes Phenyl, so sind Beispiele dafür Methylphenyl, Dimethylphenyl, Trimethylphenyl, Ethylphenyl, Diethylphenyl, Isopropylphenyl oder 6-Butylphenyl.

Beispiele für Phenylalkyl mit 7 bis 9 C-Atomen sind Benzyl, Phenethyl, $\alpha$-Methylbenzyl oder $\alpha,\alpha$-Dimethylbenzyl. Bevorzugt ist Benzyl.

Alkenyl mit bis zu 18 C-Atomen bedeutet beispielsweise Vinyl, Propenyl, Allyl, Butenyl, Methallyl, Hexenyl, Decenyl oder Heptadecenyl.

Bedeutet m = 2, so wird damit beispielsweise die Gruppe $-CH_2-CH_2-$ beschrieben, bedeutet m = 3 so werden beispielsweise die Gruppen $-CH_2-CH_2-CH_2-$ oder $-CH_2-CH(CH_3)-$beschrieben.

Bei den als Katalysatoren im erfindungsgemässen Verfahren einsetzbaren Zinn(IV)-Verbindungen handelt es sich im allgemeinen um Gemische von Verbindungen unterschiedlicher Zusammensetzung. Bevorzugt sind Verbindungen oder Gemische von Verbindungen, erhältlich durch Umsetzung von einer Verbindung der Formel:

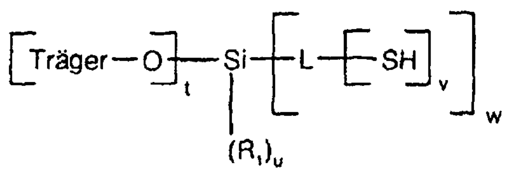

(IIa)

wobei,
t eine Zahl 1, 2, oder 3;
u eine Zahl 0, 1 oder 2;
v eine Zahl 1, 2, 3, 4, 5, 6, 7, 8 oder 9; und
w eine Zahl 1, 2 oder 3 bedeutet,
mit der Massgabe, dass t+u+w=4 ist;
L einen mindestens zweiwertigen $C_1$-$C_{18}$-Alkylen-, $C_2$-$C_{18}$-Alkenylen-, Phenylen-, $C_1$-$C_{18}$-Alkylen/Phenylen- oder durch ein oder mehrere O, C(O), S, C(S), N(Y), C(O)-N(Y), N(Y)-C(O)-N(Y) und/oder N(Y)-C(O) unterbrochenen $C_2$-$C_{18}$-Alkylen- oder $C_1$-$C_{18}$-Alkylen/Phenylen-Rest;
Träger ein anorganisches Trägermaterial;
$R_1$ unabhängig voneinander $C_1$-$C_4$-Alkoxy, Phenoxy, $C_1$-$C_4$-Alkyl, Phenyl oder Halogen; und
Y unabhängig voneinander $C_1$-$C_{18}$-Alkyl, Phenyl- oder $C_1$-$C_{18}$-Alkyl/Phenyl bedeutet; mit einer Verbindung der Formel

$$Sn[R_2]_4 \qquad (IIb),$$

wobei
$R_2$ unabhängig voneinander $C_1$-$C_{18}$-Alkyl, substituiertes $C_1$-$C_{18}$-Alkyl, $C_2$-$C_{18}$-Alkenyl, $C_1$-$C_{18}$-Halogenalkyl, $C_6$-$C_{16}$-Halogenaryl, $C_3$-$C_{16}$-Halogenheteroaryl, $C_1$-$C_4$-Alkylphenyl, $C_1$-$C_4$-Alkoxyphenyl, Halogen-$C_1$-$C_4$-alkylphenyl, Halogen-$C_1$-$C_4$-alkoxyphenyl, $C_1$-$C_{12}$-Hydroxyalkyl, $C_3$-$C_8$-Cycloalkyl, $C_6$-$C_{16}$-Aryl, substituiertes $C_6$-$C_{16}$-Aryl, $C_7$-$C_{16}$-Aralkyl, $C_3$-$C_6$-Heterocycloalkyl, $C_3$-$C_{16}$-Heteroaryl, $C_4$-$C_{16}$-Heteroaralkyl, -Cl, -Br, -I, OH, $-OC_1$-$C_{18}$-Alkyl,$-OC_6$-$C_{16}$-Aryl, -OOC-$C_1$-$C_{18}$-Alkyl, -OOC-Phenyl, SH, $-N(Si(CH_3)_3)_2$, $SC_1$-$C_{18}$-Alkyl, $-SC_6$-$C_{16}$-Aryl, -SC(O)-$C_1$-$C_{18}$-Alkyl, -SC(O) -Phenyl, -SC(S)-$C_1$-$C_{18}$-Alkyl, -SC(S)-Phenyl, H oder eine
Sauerstoffbrücke zu einem weiteren Sn Atom;
oder zwei Reste $R_2$ zusammen auch =S oder =O bedeuten,
mit der Massgabe, dass mindestens ein $R_2$ Rest vorhanden ist, welcher mit einer SH-Gruppe unter Bildung einer S-Sn Bindung reagieren kann.

L beutet bevorzugt $C_1$-$C_{18}$-Alkylen, $C_2$-$C_{18}$-Alkenylen, Phenylen oder $C_1$-$C_{18}$-Alkylen/Phenylen, besonders bevorzugt ist $C_1$-$C_{18}$-Alkylen, ganz besonders bevorzugt ist $C_2$-$C_6$-Alkylen.

Bevorzugt bedeutet $R_1$ $C_1$-$C_4$-Alkoxy, besonders bevorzugt Methoxy oder Ethoxy.

$R_2$ bedeutet bevorzugt Halogen, OH oder =O, besonders bevorzugt Chlor.

Beispiele für einen mindestens zweiwertigen $C_1$-$C_{18}$-Alkylen-Rest sind geradkettige oder verzweigte $C_1$-$C_{18}$-Alkylen-Reste der weiter unten genannten Art, bei denen mindestens ein weiteres H-Atom durch eine direkte Bindung

ersetzt ist, zum Beispiel $-CH_2-$, $-CH_2CH_2-$, $-C(CH_3)_2-$, $-CH_2C(CH_3)CH_2-$, =CH- oder $-CH_2CHCH_2-$.

Mindestens zweiwertige $C_2$-$C_{18}$-Alkenylen-Reste sind zu den beschriebenen mindestens zweiwertigen Alkylenresten analog, sie weisen eine oder mehrere C-C-Doppelbindungen auf.

Mindestens zweiwertige Phenylen-Reste sind beispielsweise o-Phenylen oder p-Phenylen.

Mindestens zweiwertige $C_1$-$C_{18}$-Alkylen/Phenylen-Reste leiten sich von den beschriebenen mindestens zweiwertigen Alkylenresten ab, indem die Alkylenkette durch Phenylen unterbrochen ist, wobei Phenylen auch den Anfang und/oder das Ende der Kette bilden kann.

Mindestens zweiwertige durch ein oder mehrere O, C(O), S, C(S), N(Y), C(O)-N(Y), N(Y)-C(O)-N(Y) und/oder N(Y)-C(O)-O unterbrochene $C_2$-$C_{18}$-Alkylen- oder $C_2$-$C_{18}$-Alkylen/Phenylen-Reste leiten sich von den beschriebenen analogen mindestens zweiwertigen Resten ab, wobei im allgemeinen bei mehreren unterbrechenden Resten diese nicht direkt benachbart sind. Beispiele für Reste dieser Art, bei denen die unterbrechenden Reste auch den Anfang und/oder das Ende der Kette bilden können, sind: $-CH_2CH_2-O-CH_2-CH_2-$, $-CH_2-C_6H_4-CH_2-O-CH_2-$, $-CH_2-CH_2-C(O)-CH_2$, $-CH_2-CH_2-C(O)$, $-CH_2-CH-CH_2-CH_2-N(C_2H_5)-CH_2-CH_2-O-$ oder $-CH_2-CH_2-CH-CH_2-O-CH_2-CH_2-O-$.

Dreiwertige Alkylenreste L sind beispielsweise

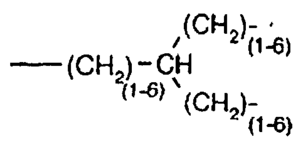

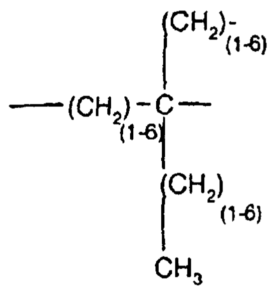 , 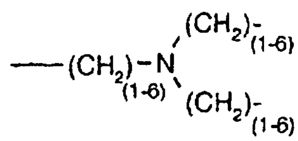 , 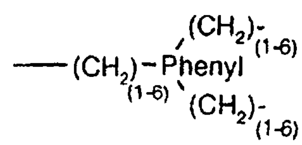

mit der Massgabe, dass die Summe der C-Atome höchstens 18 beträgt.

In den obigen Definitionen ist unter Halogen, Fluor, Chlor, Brom und Jod zu verstehen. Bei mehreren Halogensubstituenten können diese gleichartig oder gemischt (zum Beispiel Cl und F) sein.

Alkyl ist Methyl, Ethyl, Isopropyl, n-Propyl, n-Butyl, iso-Butyl, sek-Butyl, tert-Butyl sowie die verschiedenen isomeren Pentyl-, Hexyl-, Heptyl-, Octyl-, Nonyl- Decyl- Undecyl- und Dodecylradikale.

Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl, Trifluormethyl und Dichlorfluormethyl.

Alkoxy ist beispielsweise Methoxy, Ethoxy, Propyloxy, i-Propyloxy, n-Butyloxy, i-Butyloxy, s-Butyloxy und t-Butyloxy; vorzugsweise Methoxy und Ethoxy.

Halogenalkoxy ist z.B. Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, 2-Chlorethoxy und Trifluormethoxy.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl oder Cyclohexyl.

Alkoxyalkyl ist z.B.: Methoxymethyl, Ethoxymethyl, Propyloxymethyl, Methoxyethyl, Ethoxyethyl, Propyloxyethyl, Methoxypropyl, Ethoxypropyl oder Propyloxypropyl.

Phenyl, auch als Teil eines Substituenten wie Phenoxy, Phenylthio, Phenoxycarbonyl, Phenylaminocarbonyl, Benzyl oder Benzoyl, kann im allgemeinen unsubstituiert oder durch weitere Substituenten substituiert vorliegen. Die Substituenten können dann in ortho-, metaund/oder para-Stellung stehen. Bevorzugte Substituentenstellungen sind die ortho- und para-Position zur Ringverknüpfungsstelle. Bevorzugte Substituenten sind Halogenatome.

Aralkyl bedeutet z.B. Benzyl, Phenethyl, 3-Phenylpropyl, α-Methylbenzyl, Phenbutyl und α,α-Dimethylbenzyl.

Aryl und analog Halogenaryl steht beispielsweise für Phenyl, Tetralin, Inden, Naphtalin, Azulin und Anthracen.

Heteroaryl und analog Halogenheteroaryl steht beispielsweise für Pyrrol, Furan, Thiophen, Oxazol, Thiazol, Pyridin, Pyrazin, Pyrimidin, Pyridazin, Indol, Purin, Chinolin und Isochinolin. Heterocycloalkyl bedeutet z.B. Oxiran,

Oxetan, Azetidin, Azirin, 1,2-Oxathiolan, Pyrazolin, Pyrrolidin, Piperidin, Piperazin, Morpholin, Dioxolan, Telrahydropyran, Tetrahydrofuran und Tetrahydrothiophen.

Bei den anorganischen Trägermaterialien handelt es sich bevorzugt um Glas, Silikate, Halbmetall- und Metalloxide, die besonders bevorzugt als Pulver mit einem mittlereren Teilchendurchmesser von 10 nm bis 5 mm vorliegen. Es kann sich um kompakte oder poröse Teilchen handeln. Poröse Teilchen weisen bevorzugt hohe innere Oberflächen auf, zum Beispiel 1 - 1200 $m^2/g$. Weiterhin bevorzugt sind als Trägermaterialien, solche anorganischen Trägermaterialien, welche OH-Gruppen enthalten. Beispiele für Oxide und Silikate sind $SiO_2$, $TiO_2$, $ZrO_2$, MgO, NiO, $WO_3$, $Al_2O_3$, $La_2O_3$, Silicagele, Tone und Zeolithe. Besonders bevorzugte Trägermaterialien sind Silicagele, Aerosile, Aluminiumoxid, Titanoxid und deren Gemische. Ein Beispiel für Glas als Trägermaterial ist "Controlled Pore Glass", das kommerziell erhältlich ist.

Beispiele für Verbindungen enthaltend einen Rest der Formel (I) beziehungsweise für Verbindungen erhältlich durch Umsetzung von Verbindungen der Formel (IIa) und (IIb) sind:

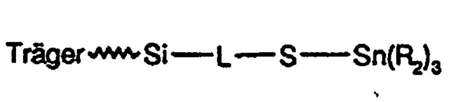 ; 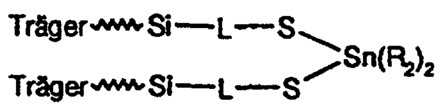 ;

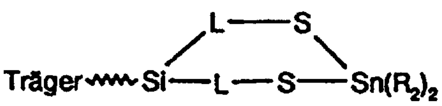 ; 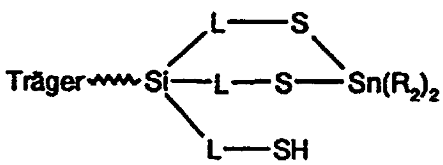 ;

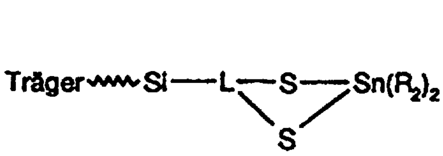 ; 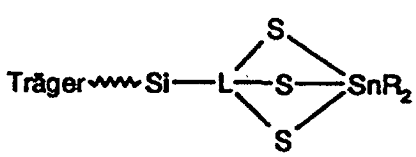 ;

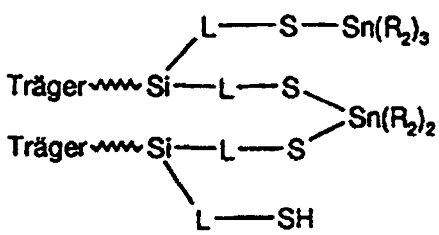 ;

wobei die Substituentenbedeutungen wie oben angegeben sind und Träger Si die Bindung zum anorganischen Trägermaterial sowie gegebenenfalls weitere an Si gebundene Reste $R_1$ bedeutet, mit der Massgabe, dass die Gesamtzahl der Bindungen am Si 4 ist.

Veranschaulichen lässt sich Träger Si durch

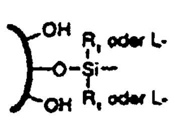 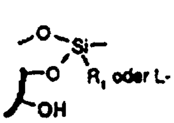 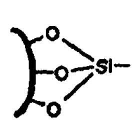 .

In der Regel wird keine einheitliche Bindung erhalten.

Die Herstellung der erfindungsgemäss verwendbaren an einen anorganischen Träger gebundenen (immobilisierten) Zinn(IV)-Verbindungen kann auf verschiedene im Prinzip bekannte Art und Weise erfolgen. Im folgenden werden zwei grundsätzliche Herstellungsmethoden beispielhaft beschrieben.

Methode 1: Es erfolgt zuerst eine Immobilisierung eines Thiols und anschliessend die Umsetzung des immobiliserten Thiols mit einer Zinn(IV)-Verbindung.

Immobilisierung des Thiols:

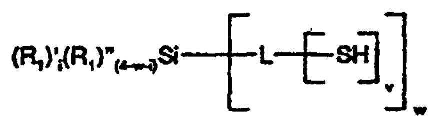

'Si-Thiol', wobei

L, v und w wie vorstehend angegeben einschliesslich der Bevorzugungen;

$R_1$' $C_1$-$C_4$-Alkyl oder Phenyl;

$R_1$" unabhängig voneinander $C_1$-$C_4$-Alkoxy, Phenoxy oder Halogen; und

i eine Zahl 0, 1 oder 2 bedeutet, mit der Massgabe, dass w+i kleiner 4 ist.

Die Immobilisierung von Alkoxy- und Halogen-Silanen auf einem anorganischen Träger ist in der Literatur eingehend beschrieben (beispielsweise durch: K.K. Unger, Porous Silica, in Journal of Chromatography Library, Vol 16, Elsevier Scientific Publishing Company (1979)). Die Belegungsdichte (mmol Thiol/g Träger) des Si-Thiol kann in einem weiten Bereich durch die Menge des eingesetzten Si-Thiol im Verhältnis zur eingesetzten Menge Träger und durch die Immobilisierungs-Bedingungen gesteuert werden. Die Si-Thiol-Verbindung wird üblicherweise in einem Lösungsmittel gelöst (z.B. Xylol, Toluol, Benzol. THF,

Methylenchlorid, Essigester oder Ether). Bevorzugt sind apolare und hochsiedende Lösungsmittel. Diese Lösung wird dann zum Träger (oder umgekehrt) gegeben und das Gemisch typischerweise 3-50 Std, bevorzugt während ca. 10 -20 Std. bei Temperaturen zwischen 50°C bis zur Rückflusstemperatur leicht gerührt. Das entstehende Wasser beziehungsweise der entstehende Alkohol wird abdestilliert. Das Produkt wird dann abfiltriert und mit einem geeigneten Lösungsmittel (z.B. Alkohol) gewaschen, um allenfalls überschüssiges Thiol zu entfernen. Das Produkt kann anschliessend bei erhöhter Temperatur bevorzugt unter Vakuum getrocknet werden oder es kann direkt, ohne Trocknen in einem für das Umsetzen mit Sn(IV)-Verbindungen geeigneten Lösungsmittel aufgeschlämmt werden. Um eine allfällige Oxidation des Thioalkohols zu entsprechenden Disulfiden zu verhindern, kann bei Bedarf unter Inertgas (z.B. Stickstoff, Argon) gearbeitet werden.

Es ist auch möglich, Thiol enthaltende Festkörper (Polysiloxane) durch Kondensation von Verbindungen 'Si-Thiol' alleine oder zusammen mit Alkoxysilanen zu erhalten.

Beispiele hierfür sind in der DE 30 29 599 gegeben (Vernetzung von Metall-Phosphin-$Si(OR)_3$ Komplexen mit $H_2O$ zu Polysiloxanen und auch Vernetzung von Phosphin-$Si(OR)_3$ zu Polysiloxanen und anschliessend Bildung von Metall-Komplexen.). Eine Übersicht findet sich beispielsweise in C. J. Brinker, G. W. Scherrer, Buch: Sol Gel Science, Academic Press (1990).

Die immobilisierten Thiole werden anschliessend mit Sn(IV) umgesetzt. (Allg. Literatur über Herstellung von Sn-Verbindungen mit Sn-S Bindungen: Gmelin, 8. Auflage, Zinn). Diese können entweder reine $Sn(R_2)_4$ Verbindungen oder Mischungen von verschiedenen $Sn(R_2)_4$ Verbindungen sein. Die 4 Reste $R_2$ können gleich oder verschieden sein. Mindestens ein $R_2$ muss ein Rest sein, welcher mit einer Thiolgruppe unter Bildung einer S-Sn Bindung reagieren kann. Verbindungen mit einem Rest $R_2$ dieser Art sind bevorzugt Halogenide, Alkoholate, Carbonate oder Sn-Verbindungen mit OH oder Sn=O Bindungen. Es ist auch bekannt, dass $Sn(Alkyl)_4$-Verbindungen mit Thiolen unter Bildung einer Sn-S Bindung reagieren können. Die Sn(IV)-Verbindung wird im allgemeinen in einem geeigneten Lösungsmittel (z.B. lineare und cyclische Alkane, Aromaten wie Benzol oder Toluol, Halogenkohlenwasserstoffe, Nitromethan, DMSO, Alkohole, Aceton, Carbonsäuren wie Essigsäure, Ester, DMF, Nitrile und auch Wasser) gelöst und die Lösung zum Trägerfixierten Si-Thiol gegeben oder umgekehrt. Die Zugabe kann bei Temperaturen zwischen-78°C und 100°C erfolgen. Bei Sn-Halogenverbindungen kann bei der Reaktion mit dem Si-Thiol H-Halogensäure freigesetzt werden. Diese kann mit Basen (zum Beispiel Alkalibase, Carbonate, Ammoniak, Amine) abgefangen werden. Nach dem Zusammengeben der Sn(IV)-Verbindung(en) und dem Si-Thiol wird das Reaktionsgemisch bei Temperaturen zwischen 20°C und Siedepunkt des Lösungsmittels langsam gerührt. Die Reaktionsdauer beträgt typischerweise 1h bis mehrere Tage. Dann wird das Produkt abfiltriert und mit Lösungsmitteln wie z.B. Alkoholen, Wasser gewaschen und bei Bedarf mit leicht basischer wässeriger Lösung oder einer den oder die Liganden modifizierenden Lösung nachbehandelt. Das Verhältnis zwischen S und Sn kann gesteuert werden, durch die Menge Sn(IV), welche im Verhältnis zur auf dem Träger immobilisierten Menge Thiol zugegeben wird. Das Verhältnis von S/Sn kann sich im Bereich 1:1 bis 10:1 bewegen.

Das Verhältnis von S/Sn kann auch nachträglich durch Coproportionierung mit einer Sn(IV) Verbindung geändert werden.

Die immobilisierten Katalysatoren können ungetrocknet eingesetzt werden, oder sie können vor Gebrauch bei erhöhter Temperatur (bevorzugt unter Vakuum) getrocknet, und bei Bedarf vor Gebrauch auch thermisch behandelt werden, beispielsweise bei Temperatur im Bereich von 100°C - 300°C.

Anstelle der Immobilisierung von Thiolen kann man auch Disulfide beispielsweise der folgenden Typen wie oben für die Thiole beschrieben immobilisieren:

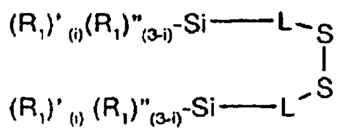 , 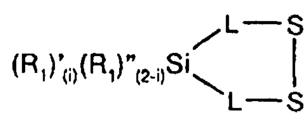 oder 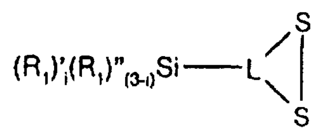

wobei die Substituentenbedeutungen wie vorstehend angegeben sind, einschliesslich ihrer Bevorzugungen und i eine Zahl 1-3 bedeutet.

Derartige Disulfide sind bekannt oder können nach bekannten Verfahren einfach hergestellt werden (W. Buder, Z. Naturforsch., 34b (1979) 790 - 793). Zum Teil sind sie im Handel erhältlich und werden beispielsweise in der Firmenschrift " Chemieforschung im Degussa Forschungszentrum Wolfgang", Band 1, Seiten 94-99, 1988 beschrieben.

Die immobilisierten Disulfide können anschliessend entweder

a) direkt mit Sn-Verbindungen umgesetzt werden:

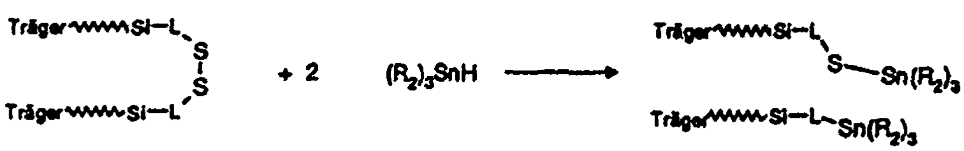

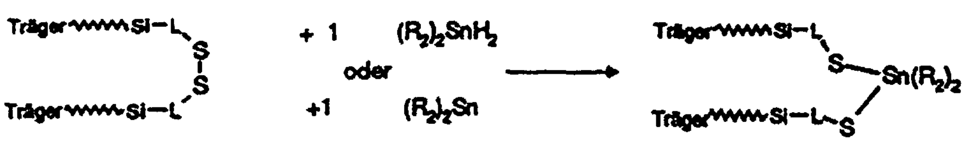


oder

b) zuerst zu Thiolen oder Thiolaten reduziert werden (z.B. mit $Na_2S$, $K_2S$, Traubenzucker, Natriumdithionit, Phosphin) und anschliessend wie für die Thiole beschrieben mit Sn(IV)-Verbindungen umgesetzt werden.

Methode 2: Zuerst Herstellung einer, gegebenenfalls isolierten, immobilisierbaren Sn-S-Verbindung und dann Immobilisierung dieser Verbindung. (Allgemeine Literatur über Herstellung von Sn-Verbindungen mit Sn-S Bindungen: Gmelin, 8. Auflage, Zinn). Reine immobilisierbare Sn-S Verbindungen oder Gemische davon werden erhalten, indem n° Verbindungen vom Typ Si-SM mit n° • v • w SM-Gruppen mit Verbindungen $Sn(R_2)_4$ teilweise oder vollständig umgesetzt werden, wobei mindestens eine Sn-S Bindung entstehen muss.

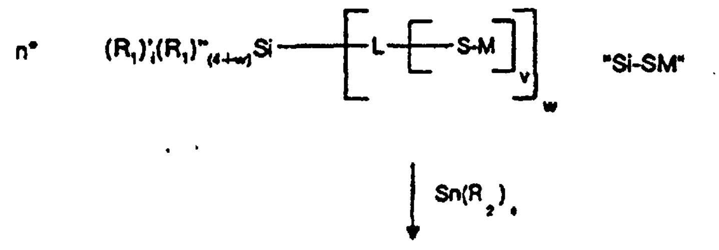


Immobilisierbare Sn-S Verbindungen

Die Bedeutung der Substituenten sowie die Indices i, v und w sind vorstehend angegeben einschliesslich ihrer Bevorzugungen, M H oder ein Alkalimetall, bevorzugt Na, K oder Li bedeutet und n° 1-4 ist, bevorzugt 1-3.

Beispiele der entstehenden Verbindungen sind nachstehend angegeben.

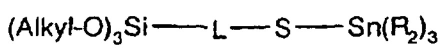 ; 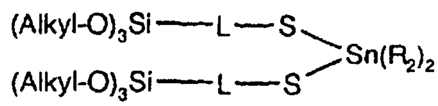 ;

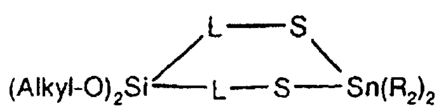 ; 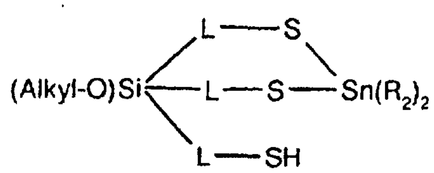 ;

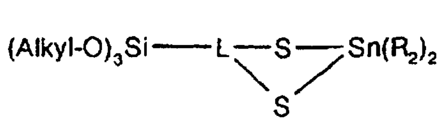 ; 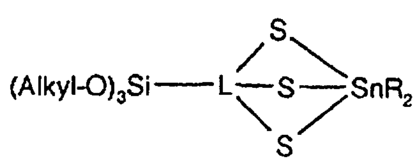 ;

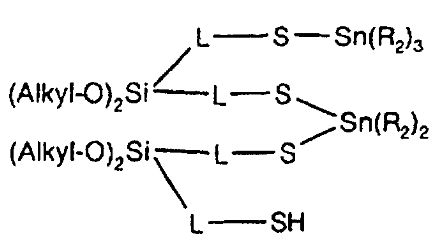

Falls mindestens ein $R_2$ Halogen bedeutet, erfolgt die Herstellung der immobilisierbaren Sn-S Verbindungen

durch Reaktion des Thiols oder eines Thiolats mit $Sn(R_2)_4$. Bei der Reaktion mit dem Thiol kann die entstehende Halogensäure mit einer Base (z.B. Alkalibasen, Carbonate, Ammoniak, Amine) abgefangen werden.

Falls mindestens ein $R_2$ O-Alkyl, O-Aryl, OH oder $N(Alkyl)_2$ oder zwei $R_2$ zusammen eine Doppelbindung zu Sauerstoff bedeutet erfolgt die Herstellung der immobilisierbaren Sn-S

Verbindungen durch Reaktion des Thiols mit $Sn(R_2)_4$. Bei Bedarf kann das während der Reaktion entstehende HO-Alkyl, HO-Aryl, $H_2O$ oder Amin abdestilliert werden.

Es ist auch bekannt, dass $Sn(Alkyl)_4$ Verbindungen mit Thiolen unter Bildung einer Sn-S Bindung reagieren können. (Gmelin)

Können mehrere $R_2$ mit dem Thiol/Thiolat reagieren, so kann durch die Wahl des Verhältnisses Thiol / Sn die bevorzugte Stöchiometrie am Sn eingestellt werden.

Die immobilisierbaren Sn-S Verbindungen können analog wie die Thiolverbindungen auf einem Träger immobilisiert oder polykondensiert werden (siehe Methode 1)

Nach der Immobilisierung können die immobilisierten Katalysatoren ungetrocknet eingesetzt werden, oder können vor Gebrauch bei erhöhter Temperatur (bevorzugt unter Vakuum) getrocknet, und bei Bedarf vor Gebrauch auch thermisch behandelt werden (bei Temperaturen im Bereich von 100°C - 300°C).

Der Katalysator kann in dispergierter Form in den Reaktanden vorliegen und wird nach erfolgter Umsetzung abfiltriert. Der durch Gebrauch erschöpfte Katalysator kann regeneriert und neu verwendet werden oder aber verworfen werden. Es ist auch möglich, den Katalysator in einem Festbett anzuordnen und die Reaktion beispielsweise in einem Strömungsreaktor durchzuführen (kontinuierliche Verfahrensweise).

Die Umesterung wird zweckmässig derart ausgeführt, dass die Ausgangsstoffe entweder in einem Lösungsmittel gelöst oder, soweit nicht flüssig, durch Temperaturerhöhung in Schmelze gebracht, umgesetzt werden.

Als Lösungsmittel kommen die an sich geläufigen Verbindungen und Gemische zur Anwendung, beispielsweise aromatische Kohlenwasserstoffe, wie Benzol, und alkylsubstituierte oder halogensubstituierte Benzole, insbesondere Toluol, Xylol oder Dichlorbenzol, hochsiedende aliphatische Kohlenwasserstoffe, wie hochsiedende Paraffine, aprotische Lösungsmittel, wie Dimethylformamid oder Dimethylanilin.

Der Katalysator wird dem Reaktionsgemisch beispielsweise in Mengen von 0,001 bis 10 Mol-%, zweckmässig von 0,01 bis 1 Mol-% aktivem Material, bezogen auf die Verbindungen enthaltend einen Rest der Formel (I), zugegeben.

Das Verhältnis von Carbonsäureester und Alkohol im Reaktionsgemisch ist nicht kritisch und kann beispielsweise 0,8 bis 1,3 Mol des Carbonsäureesters pro Aequivalent des Alkohols betragen.

Die Reaktionstemperatur kann beispielsweise zwischen 110 und 250°C und bevorzugt zwischen 130 und 210°C liegen.

Die Umesterung der Carbonsäureester bis zum Erreichen optimaler Ausbeuten dauert in der Regel zwischen 1 und 10 Stunden, zweckmässig zwischen 1 und 5 Stunden und bevorzugt zwischen 1 und 3 Stunden.

Der Katalysator kann beispielsweise in pulveriger bis stückig gebrochener Form suspendiert im Reaktionsgemisch vorliegen. Eine Anwendung des Katalysators im Festbett ist eine weitere Möglichkeit der Verfahrensführung.

Nach Beendigung der Reaktion kann der Katalysator, sofern dieser in Suspension vorlag, abgetrennt, beispielsweise abfiltriert werden und das Endprodukt durch an sich bekannte Massnahmen, wie Kristallisation aus einem Lösungsmittel, wie Methanol, Isopropanol, Methanol-Wasser-Gemisch usw., isoliert werden.

Sofern notwendig kann das Reaktionsgemisch und/oder das Endprodukt mit einer Säure, z.B. Ameisensäure, Essigsäure, Schwefelsäure, Salzsäure usw., neutralisiert werden.

Die im erfindungsgemässen Verfahren eingesetzten Carbonsäurester und Alkohole sind bekannt oder können nach an sich bekannten Verfahren erhalten werden.

Ein weiterer Gegenstand der Erfindung ist die Verwendung einer an einen anorganischen Träger gebundenen Zinn(IV)-Verbindung enthaltend einen Rest der Formel

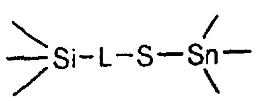 (I),

worin L ein mindestens zweiwertiger Rest ist und mindestens eine der freien Valenzen des Si in Formel (I) an den anorganischen Täger gebunden ist, als Katalysator bei der Umesterung von Carbonsäureestem.

Die erfindungsgemäss hergestellten Carbonsäureester stellen beispielsweise wertvolle Antioxidantien gegen oxidativen, thermischen oder actinischen Abbau von empfindlichen organischen Materialien dar. Solche Materialien sind beispielsweise synthetische Polymere oder funktionelle Flüssigkeiten, wie Schmierstoffe, Hydraulikflüssigkeiten oder Metallbearbeitungsflüssigkeiten etc.

Beispiele:

A) Immobilisierung von Thiolen und Disulfiden

Alle Reaktionen werden unter Inertgas durchgeführt.

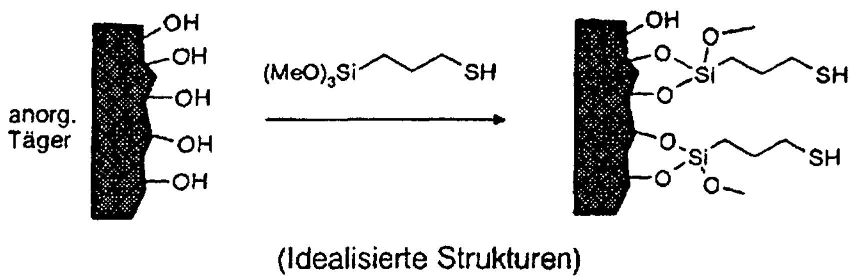


Allgemeine Vorschrift zur Immobilisierung von 3-Mercaptopropyltrimethoxysilan 100 g Träger werden in 700 ml Toluol in einer Inertgas-Atmosphäre gerührt und durch Abdestillieren von 200 - 300 ml Toluol getrocknet. Nach Abkühlen auf ca. 40°C werden 40 ml (0,215 Mol) 3-Mercaptopropyltrimethoxysilan zugegeben, das Gemisch nochmals kurz andestilliert und anschliessend bei 100° - 110°C während 20 Std. gerührt, wobei gewährleistet wird, dass das entsehende Methanol abdestillieren kann. Nach Abkühlen wird der Träger abfiltriert, mit je 2 x 200 ml Essigsäureethylester, Methanol und Hexan gewaschen und schliesslich am Vakuum (1 mbar) bei 70°C während 15 Std. getrocknet.

Beispiel A1

20 g getrocknetes Silikagel (Merck 100) (2h bei 110°C im Vakuum-Ofen) werden in einem Kolben mit Rührer in 80 ml getrocknetem Toluol suspendiert und mit 10 g (51 mmol) 3-Mercaptopropyltrimethoxysilan (Fluka, purum) versetzt. Das Gemisch wird während 12h bei 100°C gerührt und das entstehende Methanol abdestilliert. Danach wird es abfiltriert und 4 mal mit 100 ml Methanol gewaschen. Schtiesslich wird das funktionalisierte Silikagel am Vakuum bei 70-100°C getrocknet. Es wird 22,4g Produkt mit einem S-Gehalt von 2,85% erhalten. Dies entspricht einer Belegungsdichte von 0,89 mmol Thio-Verbindung pro g Material.

Die Beispiele A2-A9 werden nach der vorstehend angegebenen allgemeinen Vorschrift ergestellt, wobei die in der Tabelle 1 angegebenen Resultate bei den entsprechenden Variationen erhalten werden.

Tabelle 1

| Bsp. Nr. | Träger | Herstellung, Abweichung von Vorschrift | S-Gehalt Analytik | Belegung mMol S/g |
|---|---|---|---|---|
| A2 | Silikagel Merck 100 | Ansatz mit nur 20 g Träger. Trocknen des Silikagels nicht durch azeotrope Destillation in Toluol sondern am Vakuum (5 mbar) bei 120°C während 2 Std. Immobilisierung in 80 ml Toluol und 0,05 Mol 3-Mercaptopropyltrimethoxysilan. Waschen nur mit Methanol. | 2,85% | 0,89 |
| A3 | Silikagel Merck 100 | 0,260 Mol 3-Mercaptopropyltrimethoxysilan zugeben. Waschen nur mit Methanol. | 2,33% | 0,73 |
| A4 | Silikagel, Grace 332 | Wie Vorschrift | 1,65% | 0,52 |

Tabelle 1 (fortgesetzt)

| Bsp. Nr. | Träger | Herstellung, Abweichung von Vorschrift | S-Gehalt Analytik | Belegung mMol S/g |
|---|---|---|---|---|
| A5 | Silikagel, Grace 332 | Trocknen des Silikagels nicht durch azeotrope Destillation in Toluol sondern am Hochvakuum (0,1 mbar) bei 140°C während 4 Std. | 0,75% | 0,234 |
| A6 und A7 | Silikagel, Grace 332 | Nach Zugabe des 3-Mercaptopropyltrimethoxysilan 0,5 ml Methansulfonsäure zugeben | 2,98% | 0,93 |
| A8 und A9 | Silikagel Merck 100 | 0,269 Mol 3-Mercaptopropyltrimethoxysilan zugeben. Waschen nur mit Methanol. | 2,73% | 0,85 |

Beispiel A10

Immobilisierung von Bis(3-triethoxysilylpropyl)dilsulfid

Das Bis(3-triethoxysilylpropyl)dilsulfid wird analog wie von W. Buder, Z. Naturforsch., 34b (1979) 790 - 793 beschrieben hergestellt.

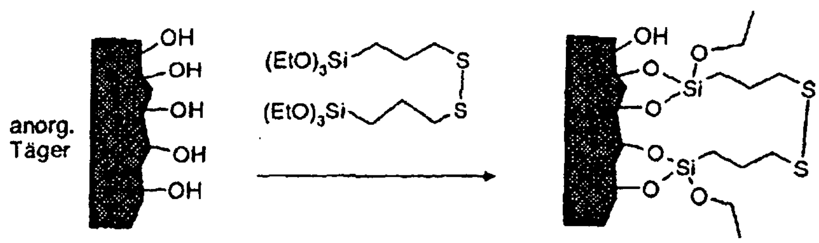

Idealisierte Strukturen

50 g Kieselgel Merck 100 werden in 250 ml Toluol in einer Inertgas-Atmosphäre gerührt und durch Abdestillieren von ca. 50 ml Toluol getrocknet. Nach Abkühlen auf ca. 40°C werden 23,7 g (0,05 Mol) Bis(3-triethoxysilylpropyl) dilsulfid zugegeben, das Gemisch nochmals kurz andestilliert und anschliessend bei 100° - 110°C während 20 Std. gerührt, wobei gewährleistet wird, dass das entsehende Ethanol abdestillieren kann. Nach Abkühlen wird der Träger abfiltriert, mit je 2 x 100 ml Essigsäureethylester, Methanol und Hexan gewaschen und schliesslich am Vakuum (1 mbar) bei 70°C während 15 Std. getrocknet. Es wird weisses Material mit einem S-Gehalt von 1,83% erhalten. Dies entspricht einer Belegungsdichte von 0,29 mMol Disulfid pro g.

B) Herstellung der Zinn(IV)-Katalvsatoren

Alle Reaktionen werden unter Inertgas durchgeführt.

Beispiel B1

8 g der Silikagel-fixierten Thio-Verbindung aus Beispiel A1 werden in 60 ml Hexan suspendiert, auf 0°C gekühlt und langsam mit 5,12 g einer 18,1% $SnCl_4$-Lösung in Hexan versetzt. Nach 15 min. werden 0,8 g Triethylamin zugegeben, und das Gemisch während 2h bei Raumtemperatur gerührt. Schliesslich wird das Produkt filtriert, zuerst mit Hexan, dann mit einer Natriumcarbonat-Lösung und mit Wasser gewaschen und dann über Nacht am Vakuum bei 80°C getrocknet. Es werden 8,1 g Produkt mit einem S-Gehalt von 2,7% und einem Sn-Gehalt von 2,46% erhalten.

Dies entspricht einer Sn-Belegungsdichte von 0,201 mmol/g und einem Verhhältnis von Sn zu S von ca 1:4.

Beispiel B2-B16

Die Ergebnisse der Umsetzung immobilisierter Schwefelverbindungen mit $SnCl_4$ sowie die Variationen der Versuchsparameter sind in Tabelle 2 zusammengefasst. (MG in kg bedeutet Gewicht des Produktes in kg pro Mol Sn)

Tabelle 2:

| Zusammenfassung der $SnCl_4$ - S Katalysatoren | | | | | | | |
|---|---|---|---|---|---|---|---|
| Kat. Nr. | Träger-SH | Träger | Bemerkungen | Analytik S% | Analytik Sn % | S/Sn | MG kg |
| B2 | A2 | Merck 100 | | 2,7 | 2,46 | 4,1 | 4,8 |
| B3 | A3 | Merck 100 | | 2,24 | 3,25 | 2,55 | 3,64 |
| B4 | A6 | Grace 332 | | 2,75 | 3,34 | 3,1 | 3,57 |
| 85 | A6 | Grace 332 | | 2,8 | 3,28 | 3,2 | 3,63 |
| B6 | A6 | Grace 332 | | 2,83 | 3,1 | 3,4 | 3,84 |
| B7 | A6 | Grace 332 | | 2,71 | 2,57 | 3,9 | 4,63 |
| B8 | A6 | Grace 332 | | 2,84 | 2,01 | 5,3 | 5,96 |
| B9 | A6 | Grace 332 | | 2,74 | 3,88 | 2,63 | 3,07 |
| B10 | A6 | Grace 332 | | 2,78 | 3,44 | 3 | 3,46 |
| B11 | A5 | Grace 332 | | 0,72 | 1,06 | 2,53 | 11,2 |
| B12 | A8 | Merck 100 | | 2,42 | 3,8 | 2,37 | 3,13 |
| B13 | Integriert | Merck 100 | | 1,89 | 2,22 | 3,2 | 5,34 |
| B14 | A7 | Grace 332 | | 2,8 | 5,13 | 2,03 | 2,33 |
| B15 | A7 | Grace 332 | | 2,8 | 4,86 | 2,15 | 2,45 |
| B16 | A6 | Grace 332 | Coproportionierung | 2,85 | 4,26 | 2,5 | 2,8 |

Et bedeutet Ethyl, Me bedeutet Methyl und Ac bedeutet Acetat.
Erläuterungen zu den Beispielen B2-B16.

**B2:** Zu einer Suspension von 8 g des immobilisierten Thiols aus Beispiel A2 (7,12 mMol S) in 65 ml wird unter Rühren eine Lösung von 0,93 g (3,56 mMol) $SnCl_4$ in 4 ml Hexan bei 0°C zugegeben. Nach 15 Min. werden 0,8 g Triethylamin zugegeben, das Gemisch 2 Std. bei Raumtemperatur gerührt, dann filtriert, zuerst mit Hexan, dann mit einer Natriumbicarbonat-Lösung und schliesslich mit viel Wasser gewaschen. Das weisse Produkt wird am Vakuum (5 mbar) bei 80°C während 15 Std. getrocknet.

**B3:** Zu einer Suspension von 100 g des immobilisierten Thiols aus Beispiel A3 (73 mMol S) in 750 ml Hexan wird unter Rühren eine Lösung von 9,5 g (36 mMol) $SnCl_4$ in 50 ml Hexan bei 0°C zugegeben. Nach 15 Min. werden 14,7 g (14,6 mMol) Triethylamin zugegeben, das Gemisch 2 Std. bei Raumtemperatur gerührt, dann filtriert. Nach dem Waschen (750 ml Hexan, 500 ml Aceton, 11 gesättigte Natriumbicarbonat-Lösung, 3l Wasser, 250 ml Ethanol) wird das weisse Produkt am Vakuum (5 mbar) bei 80°C während 15 Std. getrocknet.

**B4:** Zu einer Suspension von 10 g des immobilisierten Thiols aus Beispiel A6 (9,3 mMol S) in 50 ml Hexan wird unter Rühren eine Lösung von 2,4 g (9,3 mMol) $SnCl_4$ in 10 ml Hexan bei 0°C zugegeben. Nach 15 Min. werden 1 g (10 mMol) Triethylamin zugegeben, das Gemisch 12 Std. unter Rückfluss gerührt, abgekühlt und filtriert. Nach dem Waschen (je 50 ml: 2 x Hexan, 2 x Methanol, 2 x $NaHCO_3$ (2M), 3 x Wasser, 2 x Methanol, 2 x Essigsäureethylester, 2 x Hexan) wird das weisse Produkt am Vakuum (5 mbar) bei 80°C während 15 Std. getrocknet.

**B5:** Wird gleich wie B4 hergestellt, ausser dass nur 1,2 g (4,6 mMol) $SnCl_4$ zugegeben werden.

**B6:** Wird gleich wie B4 hergestellt, ausser dass nur 0,806 g (3,1 mMol) $SnCl_4$ zugegeben werden.

**B7:** Wird gleich wie B4 hergestellt, ausser dass nur 0,61 g (2,35 mMol) $SnCl_4$ zugegeben werden.

**B8:** Wird gleich wie B5 hergestellt, ausser dass für die Reaktion Toluol anstelle von Hexan als Lösungsmittel verwendet wird, und dass bei 100° ausreagiert wird.

**B9:** Wird gleich wie B4 hergestellt, ausser dass 2 g (20 mMol) Triethylamin verwendet werden.

**B10:** Wird gleich wie B5 hergestellt, ausser dass nur 2 Std. bei Raumtemperatur ausreagiert wird.

**B11:** Zu einer Suspension von 20 g des immobilisierten Thiols aus Beispiel A5 (4,7 mMol S) in 120 ml Toluol wird unter Rühren eine Lösung von 0,62 g (2,4 mMol) $SnCl_4$ in 10 ml Toluol bei 0°C zugegeben. Nach 15 Min. werden 0,5 g (5 mMol) Triethylamin zugegeben, das Gemisch 12 Std. bei 110°C gerührt, abgekühlt und filtriert. Nach dem Waschen (je 50 ml: 2 x Hexan, 2 x Methanol, 2 x $NaHCO_3$ (2M), 3 x Wasser, 2 x Methanol) wird das weisse Produkt am Vakuum (5 mbar) bei 80°C während 15 Std. getrocknet.

**B12:** Wird gleich wie B3 hergestellt, ausser dass die Reaktion ausgehend von 100 g immobilisierten Thiol aus Beispiel A8 (85 mMol S) in 600 ml Hexan, 42,5 mMol $SnCl_4$ in 45 ml Hexan und 1,7 Mol Triethylamin ausgeführt wird.

**B13: Integrierte Immobilisierung von Thiol und Herstellung der Sn-Verbindung.** 100 g Silikagel Merck 100 werden in 600 ml Toluol in einer Inertgas-Atmosphäre gerührt und durch Abdestillieren von 200 ml Toluol getrocknet. Nach Abkühlen auf ca. 40°C werden 25 ml (0,135 Mol) 3-Mercaptopropyltrimethoxysilan und 0,2 ml Methansulfonsäure zugegeben, das Gemisch nochmals kurz andestilliert und anschliessend bei 100° - 110°C während 20 Std. gerührt, wobei gewährleistet wird, dass das entsehende Methanol abdestillieren kann. Nach Abkühlen wird der Träger abfiltriert, und mit 2 x 200 ml Methanol gewaschen. (Eine kleine Probe wird getrocknet und analysiert → S-Gehalt 1,93%). Die Hälfte des Methanol feuchten Materials (30 mMol S) wird mit 150 ml Toluol aufgeschlämmt und langsam unter Rühren bei Raumtemperatur mit 3,9 g ( 15 mMol) $SnCl_4$ und anschliessend mit einer frisch zubereiteten Natriummethylat-Lösung in Methanol (30 mMol Natrium in 35 ml Methanol) versetzt. Das Gemisch wird 4 Std. bei Raumtemperatur gerührt, dann filtriert, gewaschen (jeweils 2 x 100ml, Methanol, $NaHCO_3$ 2M, Wasser, Methanol) und das Produkt am Vakuum (5 mbar) bei 80°C während 15 Std. getrocknet.

**B14:** Zu einer Suspension von 10 g des immobilisierten Thiols aus Beispiel A7 (9,3 mMol S) in 50 ml Methanol wird unter Rühren eine Lösung von 1,2 g (4,6 mMol) $SnCl_4$ bei Raumtemperatur zugegeben. Nach 15 Min. werden 18,6mMol Triethylamin zugegeben, das Gemisch 2 Std. gerührt, filtriert und gewaschen (je 50 ml: 2 x Wasser, 2 x $NaHCO_3$ gesättigt, 3 x Wasser, 3 x Methanol). Das weisse Produkt wird am Vakuum (5 mbar) bei 80°C während 15 Std. getrocknet.

**B15:** Wird gleich wie B14 hergestellt, ausser dass Natriumacetat anstelle von Triethylamin verwendet wird.

**B16:** Zu einer Suspension von 5 g der immobilisierten Zinnverbindung aus Beispiel B5 (4,4 mMol S, 1,38 mMol Sn) in 25 ml Toluol werden unter Rühren 0,31 g (1,2 mMol) $SnCl_4$ bei Raumtemperatur zugegeben. Das Gemisch wird dann 15 Std. bei 110°C gerührt, abgekühlt und filtriert. Nach dem Waschen (je 20 ml: 2 x Hexan, 2 x Methanol, 2 x $NaHCO_3$ (2M), 2 x Wasser, 2 x Methanol) wird das weisse Produkt am Vakuum (5 mbar) bei 70°C während 15 Std. getrocknet. Der Zinngehalt des Produktes ist 30% höher als derjenige von Beispiel B5.

Die Ergebnisse der Umsetzung von immobilisierten Alkylthiolen mit verschiedenen $S_n$(IV)-Verbindungen sowie die verschiedenen Versuchsbedingungen sind in Tabelle 3 zusammengefasst

Tabelle 3:

| Zusammenfassung verschiedene Sn(IV) Verbindungen. Beispiele B17-29 | | | | | | |
|---|---|---|---|---|---|---|
| Kat. Nr. | Träger-SH | Sn-Verb. | Analytik S % | Ana-lytik Sn % | S/Sn | MG kg |
| B17 | A4 | Bu2SnO | 1,51 | 4,11 | 1,36 | 2,9 |
| B18 | A4 | Ph2SnO | 1,48 | 3,35 | 1,65 | 3,57 |
| B19 | A4 | BuSnCl3 | 1,53 | 3,14 | 1,81 | 3,79 |
| B20 | A4 | BuSnCl3 | 1,51 | 2,68 | 2,01 | 4,44 |
| B21 | A4 | BuSnCl3 | 1,63 | 2,01 | 3,02 | 5,92 |
| B22 | A4 | PhSnCl3 | 1,46 | 2,62 | 2,1 | 4,55 |
| B23 | A4 | Bu3SnCl | 1,49 | 3,94 | 1,41 | 3,02 |
| B24 | A4 | Ph3SnOH | 1,41 | 4,46 | 1,18 | 2,57 |
| B25 | A4 | BuSnO)OH | 1,6 | 2,77 | 2,15 | 4,3 |
| B26 | A4 | 'Sn(OEt)4' | 1,6 | 3,44 | 1,72 | 3,0 |
| B27 | A6 | Bu2SnOAc | 2,72 | 4,26 | 2,37 | 2,8 |
| B28 | A6 | BuSn(OH)2Cl | 2,42 | 3,8 | 2,37 | 3,14 |
| B29 | A9 | BuSn(OH)2Cl | 2,54 | 4,36 | 2,17 | 2,75 |
| Bu bedeutet Butyl, Ph bedeutet Phenyl, B17-B28: Träger, Grace 332; B29: Träger, Merck Kieselgel 100. | | | | | | |

**B17, $Bu_2SnO$**

Zu einer Suspension von 10 g des immobilisierten Thiols aus Beispiel A4 (5,2 mMol S) in 60 ml Toluol wird unter Rühren 1 g (4 mMol) Dibutylzinnoxid zugegeben. Dann wird das Gemisch aufgeheizt und es werden 10 ml Toluol abdestilliert. Nach total 2,5 Std. wird auf Raumtemperatur abgekühlt, filtriert und gewaschen (je 50 ml: 2 x Hexan, 1 x Aceton, 3 x Wasser, 2 x Methanol, 2 x Essigsäureethylester, 2 x Hexan). Schliesslich wird das weisse Produkt am Vakuum (5 mbar) bei 50°C während 15 Std. getrocknet.

**B18, $Ph_2SnO$**

Zu einer Suspension von 10 g des immobilisierten Thiols aus Beispiel A4 (5,2 mMol S) in 60 ml Toluol wird unter Rühren 1,15 g (4 mMol) Diphenylzinnoxid zugegeben. Dann wird das Gemisch aufgeheizt und es werden 10 ml Toluol abdestilliert. Nach total 7 Std. wird auf Raumtemperatur abgekühlt, filtriert und gewaschen (je 50 ml: 2 x Hexan, 1 x Aceton, 3 x Wasser, 2 x Methanol, 2 x Hexan). Schliesslich wird das weisse Produkt am Vakuum (5 mbar) bei 50°C während 15 Std. getrocknet.

**B19, $BuSnCl_3$**

Zu einer Suspension von 10 g des immobilisierten Thiols aus Beispiel A4 (5,2 mMol S) in 40 ml Hexan wird unter Rühren eine Lösung von 1,69 g (6 mMol) Butylzinntrichlorid in 10 ml Hexan bei 0°C zugegeben. Nach 15 Min. werden 0,61 g (6 mMol) Triethylamin zugegeben, das Gemisch 3 Std. unter Rückfluss gerührt, abgekühlt und filtriert. Nach dem Waschen (je 50 ml: 2 x Hexan, 2 x Methanol, 2 x $NaHCO_3$ (2M), 3 x Wasser, 2 x Methanol, 2 x Essigsäureethylester, 2 x Hexan) wird das weisse Produkt am Vakuum (5 mbar) bei 50°C während 15 Std. getrocknet.

**B20, $BuSnCl_3$**

Gleich wie Beispiel B19 , ausser dass 0,85 g (3 mMol) Butylzinntrichlorid zugegeben werden.

**B21, $BuSnCl_3$**

Gleich wie Beispiel B19, ausser dass 0,51 g (1,8 mMol) Butylzinntrichlorid zugegeben werden.

**B22, $PhSnCl_3$**

Gleich wie Beispiel B19, ausser dass 0,94 g (3 mMol) Phenylzinntrichlorid zugegeben werden.

**B23 $Bu_3SnCl$**

Gleich wie Beispiel B19, ausser dass 1,95 g (6 mMol) Tributylzinntchlorid zugegeben werden.

**B24, $Ph_3SnOH$**

Zu einer Suspension von 10 g des immobilisierten Thiols aus Beispiel A4 (5,2 mMol S) in 40 ml Hexan wird unter Rühren eine Suspension von 2,2 g (6 mMol) Triphenylzinnhydroxid in 20 ml Hexan bei 0°C zugegeben. Das Gemisch wird andestilliert und anschliessend 16 h rückflussiert. Dann wird abgekühlt und filtriert. Nach dem Waschen (je 50 ml: 2 x Hexan, 2 x Essigsäureethylester, 2 x Methanol, 2 x Essigsäureethylester, 2 x Hexan) wird das weisse Produkt am Vakuum (5 mbar) bei 50°C während 15 Std. getrocknet.

**B25, BuSn(O)OH**

Zu einer Suspension von 10 g des immobilisierten Thiols aus Beispiel A4 (5,2 mMol S) in 70 ml Hexan werden unter Rühren festes 0,56 g (2,6 mMol) Butylzinnhydroxid-oxid bei 0°C zugegeben. Das Gemisch wird andestilliert und anschliessend 16 h rückflussiert. Dann wird abgekühlt und filtriert. Nach dem Waschen (je 50 ml: 2 x Essigsäureethylester, 2 x Methanol, 2 x Essigsäureethylester, 2 x Hexan) wird das weisse Produkt am Vakuum (5 mbar) bei 60°C während 15 Std. getrocknet.

**B26, $SnCl_4$/NaOEt**

10 mMol frisch ausgehend von Natrium und Ethanol hergestelltes Natriumethylat werden in 50 ml Toluol aufgeschlämmt. Dann werden bei 0°C 0,65 g (2,5 mMol) Zinntetrachlorid gegeben. Zur resultierenden Lösung werden bei Raumtemperatur 10 g des immobilisierten Thiols aus Beispiel A4 (5,2 mMol S) gegeben, das Gemisch andestilliert und 3 Std. rückflussiert. Nach dem Waschen (je 50 ml: 2 x Wasser, 2 x Methanol, 2 x Essigsäureethylester, 2 x Hexan) wird das weisse Produkt am Vakuum (5 mbar) bei 60°C während 15 Std. getrocknet.

**B27, $Bu_2Sn(OAc)_2$**

Zu einer Suspension von 10 g des immobilisierten Thiols aus Beispiel A6 (9,3 mMol S) in 60 ml Toluol wird unter Rühren 1,51 g (4,3 mMol) Dibutylzinndiacetat bei 0°C zugegeben. Dann wird das Gemisch aufgeheizt, es werden 15 ml Toluol abdestilliert und anschliessend wird während 15 Std. bei 110°C gerührt. Dann wird auf Raumtemperatur abgekühlt, filtriert und gewaschen (je 50 ml: 2 x Hexan, 1 x Aceton, 2 x Methanol, 2 x Wasser, 2 x Methanol, 2 x Essigsäureethylester, 2 x Hexan) und das weisse Produkt am Vakuum (5 mbar) bei 70°C während 15 Std. getrocknet.

**B28, $BuSn(OH)_2Cl$**

Gleich wie B27, ausser dass 1,2 g Butylzinnchlorid-dihydroxid zugegeben werden und wie folgt gewaschen wird: je 50 ml, 2 x Methanol, 2 x Essigsäureethylester, 2 x Hexan.

**B29, $BuSn(OH)_2Cl$**

Zu einer Suspension von 50 g des immobilisierten Thiols aus Beispiel A9 (42 mMol S) in 300 ml Toluol wird unter Rühren 5,15 g (21 mMol) Butylzinnchlorid-dihydroxid bei Raumtemperatur zugegeben. Dann wird das Gemisch aufgeheizt, es werden ca. 40 ml Toluol abdestilliert und anschliessend während 15 Std. bei 110°C gerührt. Dann wird auf Raumtemperatur abgekühlt, filtriert und gewaschen (je 250 ml: 3 x Methanol, 2 x Essigsäureethylester, 2 x Hexan) und das weisse Produkt am Vakuum (5 mbar) bei 70°C während 15 Std. getrocknet.

<u>Immobilisierung von Sn(IV)-S Verbindungen mit Trialkoxysilan-Resten</u>

Herstellung der Sn(IV)-S Verbindungen mit Trialkoxysilan-Resten

**B30** Herstellung von $(Bu)_2Sn[S(CH_2)_3Si(OCH_3)_3]_2$

$$2\ HS(CH_2)_3Si(OCH_3)_3 + (Bu)_2SnCl_2 + 2\ (C_2H_5)_3N \rightarrow (Bu)_2Sn[S(CH_2)_3Si(OCH_3)_3]_2 + 2\ (C_2H_5)_3N{*}HCl$$

Es werden 98,2 g (0,50 mol) 3-Mercaptopropyl-trimethoxysilan und 52,6 g (0,52 mol) Triethylamin in 200 ml n-Hexan vorgelegt. Anschliessend wird eine Lösung aus 76,0 g (0,25 mol) Dibutylzinndichlorid in 300 ml n-Hexan während ca. 20 min. zudosiert und die Vorlage mit 200 ml n-Hexan gespült. Die Innentemperatur steigt dabei von 20 auf 35°C an. Gleich bei Beginn der Dosierung fällt ein weisser Niederschlag aus. Nach 24 h Ausrühren bei ca. 20°C wird der Niederschlag abfiltriert, das Filtrat und die Rückstände mit 200 ml n-Hexan gewaschen. Die noch leicht trüben Filtrate werden vereinigt und in einem Doppelmantelreaktor bis zu einer Temperatur von ca. 61°C und 1 hPa eingedampft, wobei die Trübung verschwindet.
Bei 20°C werden ca. 143 g leicht gelbes, flüssiges Produkt abgelassen (91,8% d. Theorie). Mikroanalyse: S 10,44%, Sn 19,2%.

**B31** Herstellung von $(Bu)_2SnCl[S(CH_2)_3Si(OCH_3)_3]$

$$(Bu)_2Sn[S(CH_2)_3Si(OCH_3)_3]_2 + (Bu)_2SnCl_2 \rightarrow 2\ (Bu)_2SnCl[S(CH_2)_3Si(OCH_3)_3]$$

In einem Probeglas werden 24,3 g (0,080 mol) Dibutylzinndichlorid in 49,9 g (0,080 mol) der Verbindung aus

Beispiel B30 unter leichtem Erwärmen (ca. 50°C) gelöst und 2 Tage bei Raumtemperatur stehen lassen. Mikroanalyse: S 6,29%, Sn 26,5%

**B32** Herstellung von $(Ph)_2Sn[S(CH_2)_3Si(OCH_3)_3]_2$

$$2\ HS(CH_2)_3Si(OCH_3)_3 + (Ph)_2SnCl_2 + 2\ (C_2H_5)_3N \rightarrow (Ph)_2Sn[S(CH_2)_3Si(OCH_3)_3]_2 + 2\ (C_2H_5)_3N{*}HCl$$

Herstellung analog Beispiel B30 ausser dass 86 g (0,25 Mol) Diphenylzinndichlorid anstelle von Dibutylzinndichlorid eingesetzt werden. Das flüssige Produkt wird mit einer Ausbeute von 87,5% erhalten. Mikroanalyse: S 10,12%, Sn 17,1 %

**B33** Herstellung von $Sn[S(CH_2)_3Si(OCH_3)_3]_4$

$$4\ HS(CH_2)_3Si(OCH_3)_3 + SnCl_4 + 4\ (C_2H_5)_3N \rightarrow Sn[S(CH_2)_3Si(OCH_3)_3]_4 + 4\ (C_2H_5)_3N{*}HCl$$

Es werden 98,2 g (0,50 mol) 3-Mercaptopropyl-trimethoxysilan und 50,6 g (0,50 mol) Triethylamin in 200 ml n-Hexan vorgelegt. Anschliessend wird eine Lösung aus 32,6 g (0,125 mol) Zinntetrachlorid in 250 ml n-Hexan während ca. 45 min. zudosiert. Weiteres Vorgehen gleich wie in Beispiel B30. Das flüssige Produkt wird mit einer Ausbeute von 85,1% erhalten. Mikroanalyse: S 12,2% %, Sn 14,5 %

**B34** Herstellung von $SnCl_{4-n}[S(CH_2)_3Si(OCH_3)_3]_n$

$$Sn[S(CH_2)_3Si(OCH_3)_3]_4 + SnCl_4 \rightarrow 2\ SnCl_{4-n}[S(CH_2)_3Si(OCH_3)_3]_n$$

In ein mit Argon überlagertes Probeglas werden 40,0 g (0,044 mol) der Verbindung aus Beispiel B33 vorgelegt und 11,6 g (0,044 mol) Zinntetrachlorid eingerührt. Die Reaktion ist exotherm und es fällt ein weißer Feststoff aus. Mikroanalyse: flüssiger Anteil des Gemisches S 12,3%, Sn 14,5%; fester Anteil S 10,24%, Sn 21,8%.

**B35 :** Immobilisierung der Verbindung aus Beispiel B30 1412/21

$$(Bu)_2Sn[S(CH_2)_3Si(OCH_3)_3]_2$$

Eine Suspension von 40 g Silikagel Merck 100 in 300 ml Toluol wird zunächst durch azeotrope Destillation von 100 ml Toluol getrocknet. Nach Abkühlen auf Raumtemperatur werden 31,2 g der Zinnverbindung 1412/21 zugegeben, das Gemisch andestilliert und 18 Std. bei 100°C gerührt, wobei gewährleistet wird, dass der entstehende Alkohol abdestillieren kann. Nach Abkühlen wird das Produkt filtriert, gewaschen (je 100 ml, 3 x Methanol, 3 x Essigsäureethylester, 3 x Hexan) und am Vakuum (5 mbar) bei 70°C während 15 Std. getrocknet.

**B36** : Immobilisierung der Verbindung aus Beispiel B32 $(Ph)_2Sn[S(CH_2)_3Si(OCH_3)_3]_2$.

Wird gleich wie in Beispiel B35 hergestellt, ausser dass die Reaktion mit 33,7 g der Sn-Verbindung aus Beispiel B32 durchgeführt wird.

**B37** : Immobilisierung der Verbindung aus Beispiel B31 $(Bu)_2SnCl[S(CH_2)_3Si(OCH_3)_3]$.

Wird gleich wie in Beispiel B35 hergestellt, ausser dass die Reaktion mit 23,1 g der Sn-Verbindung aus Beispiel

B31 durchgeführt wird und dass das Waschen wie folgt erfolgt: je 100 ml, 3 x Methanol, 1 x $NaHCO_3$ 1M, 3 x Wasser, 3 x Methanol, 2 x Essigsäureethylester, 2 x Hexan.

**B38** : Immobilisierung der Verbindung aus Beispiel B34 $SnCl_{4-n}[S(CH_2)_3Si(OCH_3)_3]_n$.

Wird gleich wie in Beispiel B35 hergestellt, ausser dass die Reaktion mit 29 g der Sn-Verbindung B34 durchgeführt wird und dass das Waschen wie folgt erfolgt: je 100 ml, 3 x Methanol, 1 x $NaHCO_3$ 1 M, 3 x Wasser, 3 x Methanol, 2 x Essigsäureethylester, 2 x Hexan.
Die Ergebnisse sind in der Tabelle 4 zusammengefasst.

Tabelle 4:

| Immobilisierung von Sn(IV)-S Verbindungen mit Trialkoxysilan-Resten | | | | | | | |
|---|---|---|---|---|---|---|---|
| Kat. Nr. | Träger | Sn-Verb. Nr | Sn-Verb. | Analytik S % | Analytik Sn % | S/Sn | MG kg |
| B35 | Merck 100 | B30 | $((MeO)_3Si\text{-}C_3H_6S)_2SnBu_2$ | 1,94 | 4,2 | 1,72 | 2,84 |
| B36 | Merck 100 | B32 | $((MeO)_3Si\text{-}C_3H_6\text{-}S)_2SnPh_2$ | 2,36 | 3,86 | 2,28 | 3,01 |
| B37 | Merck 100 | B31 | $((MeO)_3Si\text{-}C_3H_6S)Sn(Bu)_2Cl$ | 2,16 | 4,4 | 1,82 | 2.71 |
| B38 | Merck 100 | B34 B | $((MeO)_3Si\text{-}C_3H_6S)_nSnCl_{4-n}$ | 4,58 | 3,48 | 4,9 | 3,35 |
| Me bedeutet Methyl, Ph bedeutet Phenyl und Bu bedeutet Butyl | | | | | | | |

Herstellung von Sn-S Verbindungen über Sol-Gel Verfahren

Sol-GeVerfahren sind beispielsweise in folgenden Publikationen beschrieben:
C. J. Brinker, G. W. Scherrer, Buch: Sol Gel Science, Academic Press (1990).
L L. Hench and J. K. West, Chem. Rev., 90 (1990) 33-72.
U. Schubert et al., J. Non Cryst. Solids, 105 (1988) 165-170.
P. Panster, CLB Chemie in Labor und Biotechnik, 43 (1992) 16-21.
Diese Verfahren können analog für die Immobilisierung der erfindungsgemässen Katalysatoren verwendet werden. Die Beispiele B39 und 40 veranschaulichen dies.

**B39**

5,42 g (6 mMol) $((CH_3O)_3Si\text{-}C_3H_6\text{-}S)_4Sn$ (Verbindung aus Beispiel B33 ), 7,3 g (48 mMol) Tetramethoxysilan, 1,56 g (6 mMol) Zinntetrachlorid und 100 ml Aceton werden in einem Kolben unter Rühren zusammengegeben. Zu dieser Lösung werden innerhalb 30 Min. 34 ml einer 2,8 n Phosphorsäure Lösung zugetropft. Diese Lösung wird in einem offenen Gefäss während 4 Tagen stehengelassen, wobei das Aceton verdampft und eine gelblichweisse Masse entsteht. Diese wird während 14 Std. in 200 ml Wasser gerührt, abfiltriert, gewaschen (je 30 ml, 3 x Methanol, 1 x $NaHCO_3$ 2M, 3 x Wasser, 3 x Methanol, 2 x Essigsäureethylester, 2 x Hexan) und am Vakuum (5 mbar) bei 70°C während 15 Std. getrocknet.

**B40**

Zu einer Lösung von 7,92 g (52 mMol) Tetramethoxysilan, 0,94 g (4 mMol) n-Octyltrimethoxysilan, 2,75 g (14 mMol) 3- Mercaptopropyltrimethoxysilan, 100 ml Aceton und 1,82 g (7 mMol) Zinntetrachlorid werden 2,83 g Triethylamin gegeben, wobei ein weisser Niederschlag entsteht. Zu diesem Gemisch werden innerhalb 30 Min. 35 ml einer 2,8 n Phosphorsäure Lösung zugetropft. Die resultierende klare Lösung wird in einem offenen Gefäss während 6 Tagen stehengelassen, wobei das Aceton verdampft und eine gelblichweisse Masse entsteht. Diese wird über Nacht bei 70°C und Vakuum (5 mbar) etwas getrocknet, gewaschen (je 50 ml, 3 x Methanol, 1 x $NaHCO_3$ 2M, 3 x Wasser, 3 x Methanol, 2 x Essigsäureethylester, 2 x Hexan) und am Vakuum (5 mbar) bei 70°C während 15 Std. getrocknet.
Die Ergebnisse sind in Tabelle 5 zusammengefasst.

Tabelle 5:

| Durch Sol Gel Verfahren hergestellte Sn-Katalysatoren: | | | | | |
|---|---|---|---|---|---|
| Kat. Nr. | Zusammensetzung | Analytik S % | Analytik Sn % | S/Sn | MG kg |
| B39 | $((MeO)_3Si\text{-}C_3H_6\text{-}S)_4Sn$, $SnCl_4$, $(MeO)_4Si$ | 6,62 | 10,4 | 2,64 | 1,28 |
| B40 | $(MeO)_3Si\text{-}C_3H_6\text{-}SH$, $SnCl_4$, $(MeO)_4Si$, n-Octyl-Si $(OMe)_3$ | 5,1 | 7,45 | 2,55 | 1,6 |
| Me bedeutet Methyl | | | | | |

Immobilisierung von Sn-S Verbindungen auf Träger-gebundene Disulfiden

**B41**

8g eines wie in der Literatur beschrieben (U. Deschler et al., Angew. Chemie., 98 (1986) 237 - 53; P. Panster, CLB Chemie in Labor und Biotechnik, 43 (1992) 16-21) durch Co-Polykondensation von Bis(3-trialkoxysilylpropyl) dilsulfid hergestellten Materials (Deloxan® DSP der Fa. Degussa) mit einem Schwefelgehalt von 19,7% werden in 50 ml Toluol durch azeotrope Destillation von 20 ml Toluol getrocknet. Nach Abkühlen wird bei Raumtemperatur eine Lösung von 1,68 g (3,69 mMol) $Sn(N(SiCH_3)_2)_2$ in 15 ml Toluol zugetropft. Dann wird das Gemisch 15 Std. auf 70°C und noch 2 Std auf 120°C gerührt. Nach Abkühlen auf Raumtemperatur wird es filtriert, gewaschen (je 30 ml, 3 x Methanol, 1 x $NaHCO_3$ 2M, 3 x Wasser, 3 x Methanol, 2 x Essigsäureethylester, 2 x Hexan) und am Vakuum (5 mbar) bei 70°C während 15 Std. getrocknet.

**B42**

10 g des auf Silikagel gebundenen Disulfids aus Beispiel A10 in 70 ml Toluol werden durch azeotrope Destillation von 40 ml Toluol getrocknet. Nach Abkühlen wird bei Raumtemperatur eine Lösung von 1,26 g (2,85 mMol) Sn $(N(SiCH_3)_2)_2$ in 10 ml Toluol zugetropft. Das weitere Vorgehen ist analog zu Beispiel B41.

**B43**

Reduktion des Disulfids zu Thiol: Zu 16 g des in PU732.1 beschriebenen Disulfids werden 90 ml Methanol, 10 ml 1,4-Dioxan, 10 ml Wasser, 5 g Triphenylphosphin und 1 ml Methansulfonsäure gegeben und das Gemisch bei 70°C während 17 Std. gerührt. Anschliessend wird das Produkt abfiltriert und mit 700 ml Methanol / Toluol gewaschen. Umsetzung mit $SnCl_4$: Zur Hälfte des feuchten Produkts in 30 ml Methanol werden unter Rühren 0.74 g Natriumacetat und anschliessend langsam 1.04 g Zinntetrachlorid gegeben. Nach 2 Std. wird das Gemisch filtriert und gewaschen, (je 40 ml, 1 x MeOH, 1 x $H_2O$, 2 x $NaHCO_3$ 2M, 3 x Wasser, 3 x MeOH) und am Vakuum (5 mbar) bei 70°C während 15 Std. getrocknet.

Tabelle 6:

| Sn Katalysatoren auf der Basis von immobilisierten Dialkyldisulfiden: | | | | | | | |
|---|---|---|---|---|---|---|---|
| Kat. Nr. | Träger | Immob. Disulfid | Sn-Verb. | Analytik S % | Analytik Sn % | S/Sn | MG kg |
| B41 | Deloxan | DSP | $Sn(N(SiCH_3)_2)_2$ | 18,39 | 4,28 | 16 | 2,8 |
| 842 | Merck 100 | A10 | $Sn(N(SiCH_3)_2)_2$ | 1,64 | 2,86 | 2,14 | 4,17 |
| B43 | Deloxan reduziert | DSP | $SnCl_4$ | 17,93 | 5,34 | 12,5 | 2,23 |

C) Anwendungsbeispiele

**Beispiel C1:**

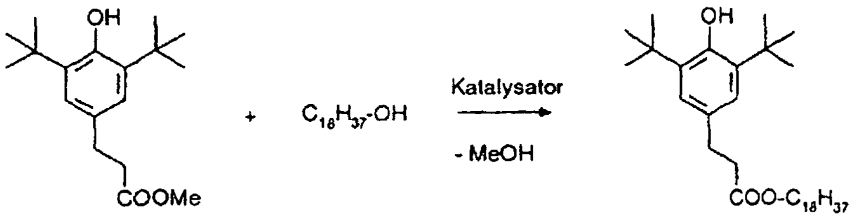


(101) (102)

17,5 g Verbindung (101) (60 mmol) und 13,5g Stearylalkohol (50 mmol) werden bei 120°C in einem Rotationsverdampfer aufgeschmolzen. Dann werden 2g des Katalysators aus Beispiel B1 zugegeben und unter Vakuum (100 mbar) die Temperatur innerhalb von 30 min. auf 190°C erhöht. Dann wird das Vakuum innerhalb 1h20' auf 8 mbar verbessert. Nach weiteren 30 min. wird dann die Schmelze filtriert und der Katalysator nochmals eingesetzt. In 4 Katalsator-Einsätzen wird laut GC jeweils ein Umsatz von 85-90% erhalten und das Produkt enthält jeweils weniger als 1 ppm Sn

**Beispiel C2**

142,6 g (0,489 mol) der Verbindung (101) und 120,35 g (0,445 mol) Stearylalkohol werden in einem Sulfierkolben vorgelegt und unter Inertgas auf 180°C aufgeheizt. Zu dieser Schmelze werden 15,39 g Katalysator aus Beispiel B3 zugegeben. Die Apparatur wird auf 3 mbar evakuiert und 4 Std. bei 180°C gerührt und das entstehende Methanol kontinuierlich abdestilliert. Anschliessend wird das Gemisch auf 150°C abgekühlt und filtriert. Wiedereinsatz: Der ungewaschene Katalysator kann wie oben beschrieben wieder eingesetzt werden.

Resultate:

| Katalysatoreinsatz | Umsatz bezogen auf eingesetzten Stearylalkohol (GC) | ppm Zinn in Rohprodukt |
|---|---|---|
| frischer Kat. | 76,8% | 2 |
| 1. Wiedereinsatz | 95,8% | <1 |
| 2. Wiedereinsatz | 98,2% | 1 |
| 3. Wiedereinsatz | 99,6% | <1 |
| 4. Wiedereinsatz | 99,9% | <1 |
| 5. Wiedereinsatz | 99,5% | <1 |

Die Selektivität ist in allen Fällen grösser als 99%. Die Transmission bei 425 nm liegt bei allen Wiedereinsätzen über 99%.

Der Katalysator ist auch nach dem 5. Wiedereinsatz noch aktiv.

**Beispiel C3**

Das Vorgehen ist gleich wie in Beispiel C2 beschrieben. Ansatzgrösse: 30,2 g (0,111 mol) Verbindung (101), 25,6 g (0,101 mol) Stearylalkohol, 2,79g Katalysator aus Beispiel B12.

Resultate:

| Katalysatoreinsatz | Umsatz bezogen auf eingesetztes Stearylalkohol (GC) | ppm Zinn in Rohprodukt |
|---|---|---|
| frischer Kat. | 61,4% | 2 |
| 1. Wiedereinsatz | 89,9% | 1 |

(fortgesetzt)

| Katalysatoreinsatz | Umsatz bezogen auf eingesetztes Stearylalkohol (GC) | ppm Zinn in Rohprodukt |
|---|---|---|
| 2. Wiedereinsatz | 98,6% | <1 |
| 3. Wiedereinsatz | 95,6% | 1 |
| 4. Wiedereinsatz | 97,5% | 2 |
| 5. Wiedereinsatz | 97,5% | <1 |

Die Selektivität ist in allen Fällen grösser als 99%. Die Transmission bei 425 nm liegt bei allen Wiedereinsätzen über 99%.

Der Katalysator ist auch nach dem 5. Wiedereinsatz noch aktiv.

**Beispiel C4**

Das Vorgehen ist gleich wie mit Mü19. Ansatzgrösse: 30,2 g (0,111 mol) Verbindung (101), 25,6 g (0,101 mol) Stearylalkohol, 1,28 g Katalysator aus Beispiel B17.

Resultate:

| Katalysatoreinsatz | Umsatz bezogen auf eingesetzten Stearylalkohol (GC) | ppm Zinn in Rohprodukt |
|---|---|---|
| frischer Katalysator | 75,5% | 8 |
| 1. Wiedereinsatz | 91,1% | 5 |

Die Selektivität ist in allen Fällen grösser als 99%. Die Transmission bei 425 nm liegt beim Wiedereinsatz über 99%.

Der Katalysator ist auch nach dem 1. Wiedereinsatz noch aktiv.

**Beispiel C5**

Das Vorgehen ist gleich wie in Beispiel C2 beschrieben. Ansatzgrösse: 33 g (0,12 mol) Verbindung (1019), 25,5 g (0,101 mol) Stearylalkohol, 2,18 g Katalysator aus Beispiel B20.

Resultate:

| Katalysatoreinsatz | Umsatz bezogen auf eingesetzten Stearylalkohol (GC) | ppm Zinn in Rohprodukt |
|---|---|---|
| frischer Kat. | 87,5% | 8 |
| 1. Wiedereinsatz | 78,5% | 10 |

Die Selektivität ist in allen Fällen grösser als 99%. Die Transmission bei 425 über 96%.

**Beispiel C6**

Das Vorgehen ist gleich wie in Beispiel C2 beschrieben. Ansatzgrösse: 30,2 g (0,111 mol) Verbindung (101), 25,6 g (0,101 mol) Stearylalkohol, 1,26 g Katalysator aus Beispiel B28.

Resultate:

| Katalysatoreinsatz | Umsatz bezogen auf eingesetzten Stearylalkohol (GC) | ppm Zinn in Rohprodukt |
|---|---|---|
| frischer Kat. | 98,7% | 4 |
| 1. Wiedereinsatz | 99,4% | <2 |

Die Selektivität ist in allen Fällen grösser als 99%. Die Transmission bei 425 nm liegt beim Wiedereinsatz über 99%.

Der Katalysator ist auch nach dem 1. Wiedereinsatz noch aktiv.

**Beispiel C7**

Das Vorgehen ist gleich wie in Beispiel C2 beschrieben. Ansatzgrösse: 30,2 g (0,111 mol) Verbindung (101), 25,6 g (0,101 mol) Stearylalkohol, 1,1 g Katalysator aus Beispiel B25. Resultate:

| Katalysatoreinsatz | Umsatz bezogen auf eingesetzten Stearylalkohol (GC) | ppm Zinn in Rohprodukt |
|---|---|---|
| frischer Kat. | 62,8% | 2 |
| 1. Wiedereinsatz | 82,1% | 1 |

Die Selektivität ist in allen Fällen grösser als 99%. Die Transmission bei 425 nm liegt beim Wiedereinsatz über 99%.

Der Katalysator ist auch nach dem 1. Wiedereinsatz noch aktiv.

**Beispiel C8**

Das Vorgehen ist gleich wie in Beispiel C2 beschrieben. Ansatzgrösse: 129,7 g (0,445 mol) Verbindung (101), 120,4 g (0,445 mol) Stearylalkohol, 2,974 g Katalysator aus Beispiel B35

Resultate:

| Katalysatoreinsatz | Umsatz bezogen auf eingesetzten Stearylalkohol (GC) | ppm Zinn in Rohprodukt |
|---|---|---|
| frischer Kat. | 92,9% | 28 |
| 1. Wiedereinsatz | 96% | 17 |

Die Selektivität ist in allen Fällen grösser als 99%. Die Transmission bei 425 nm liegt beim Wiedereinsatz über 99%.

Der Katalysator ist auch nach dem 1. Wiedereinsatz noch aktiv.

**Beispiel C9**

Das Vorgehen ist gleich wie in Beispiel C2 beschrieben. Ansatzgrösse: 30,2 g (0,111 mol) Verbindung (101), 25,6 g (0,101 mol) Stearylalkohol, 1,24 g Katalysator aus Beispiel B41. Resultate:

| Katalysatoreinsatz | Umsatz bezogen auf eingesetzten Stearylalkohol (GC) | ppm Zinn in Rohprodukt |
|---|---|---|
| frischer Kat. | 99,7% | <1 |
| 1. Wiedereinsatz | 98,5% | 1 |

Die Selektivität ist in allen Fällen grösser als 99%. Die Transmission bei 425 nm liegt beim Wiedereinsatz über 99%.

Der Katalysator ist auch nach dem 1. Wiedereinsatz noch aktiv.

**Beispiel C10**

Das Vorgehen ist gleich wie in Beispiel C2 beschrieben. Ansatzgrösse: 30,2 g (0,111 mol) Verbindung (101), 25,6 g (0,101 mol) Stearylalkohol, 3,72 g Katalysator aus Beispiel B42. Resultate:

| Katalysatoreinsatz | Umsatz bezogen auf eingesetztes Stearylalkohol (GC) | ppm Zinn in Rohprodukt |
|---|---|---|
| frischer Kat. | 85,2% | 5 |
| 1. Wiedereinsatz | 86,7% | 5 |

Die Selektivität ist in allen Fällen grösser als 99%. Die Transmission bei 425 nm liegt über 98%.

Der Katalysator ist auch nach dem 1. Wiedereinsatz noch aktiv.

**Beispiel C11**

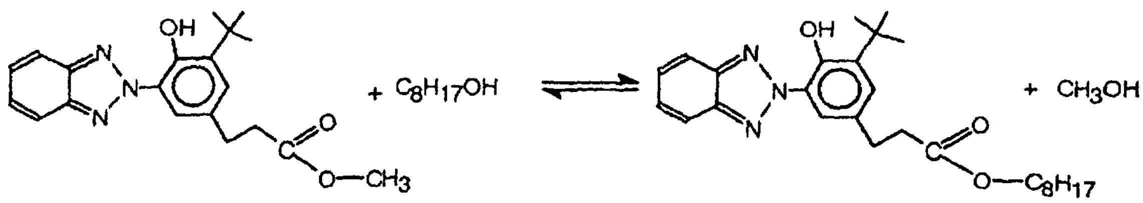

(103) Isooctanol (104) Methanol

Der in Beispiel B3 beschriebene Katalysator wird für vorstehende Umesterung eingesetzt. 4,0 g Katalysator, 100 g Verbindung (103) und 73,7 g Isooctanol Isomerengemisch werden in einem gerührten Glasreaktor mit auf 80°C beheiztem Rücklaufkühler und Vakuumpumpe vorgelegt. Der Reaktor wird anschliessend auf 400 mbar evakuiert und die Reaktionsmischung unter Rühren auf Rückflusstemperatur erhitzt. Das durch die Reaktion entstehende Methanol wird durch den auf 80°C beheizten Rückflusskühler abdestilliert und in einer nachgeschalteten Kühlfalle kondensiert. Nach ca. 4 Stunden wird eine Siedetemperatur des Reaktionsgemisches von 180-185°C erreicht. Nach weiteren 3 Stunden der Reaktion wird ein Umsatz von 99,8% bei einer Selektivität von nahezu 100% bezügl. des vorgelegten Methylesters erreicht. Nach Abkühlen der Reaktionsmasse auf 120°C wird der Katalysator abfiltriert und in den nächsten 2. Reaktionsansätzen wieder eingesetzt. Dabei wird der abfiltrierte, ungewaschene Katalysator wieder mit 100 g Methylester, Verbindung (103) und 73,7 g Isooctanol gemischt und die Reaktion nach der obigen Vorgehensweise durchgeführt. Ein Umsatz von 99,6% wird bereits nach 5 Stunden erreicht. Die Selektivität ist ebenfalls nahezu 100%. Der Restgehalt an Zinn im Produkt beträgt sowohl beim ersten als auch bei den 2 Rezyklierungsversuchen 30 ppm.

**Beispiel C12**

Herstellung von Tetrakis-[3-(3,5-di-tert-butyl-4-hydroxyphenyl)propionyloxymethyl]methane Verbindung (105)

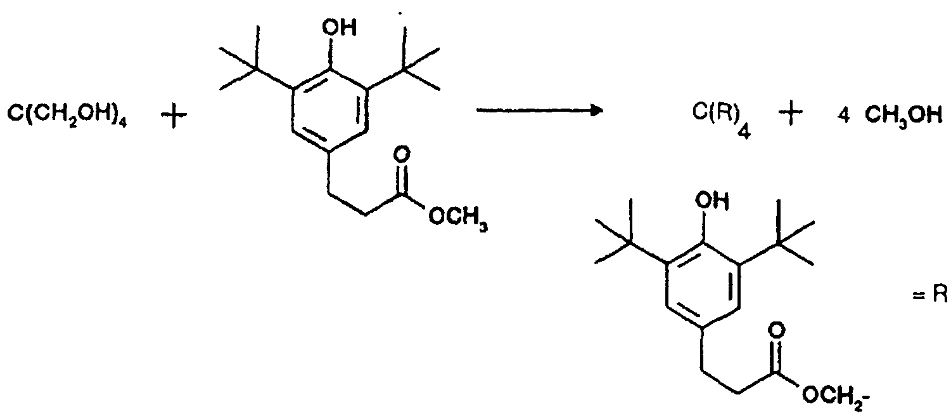

(101) (105)

250,0 g (0,856 Mole) 3,5-Bis(1,1-dimethyl)-4-hydroxyphenylpropionsäure-methylester (101), 23,3 g (0,171 mole) und 5,12 g Katalysator aus Beispiel B29 werden auf 180 °C erhitzt und für 6,5 Stunden auf dieser Temperatur gehalten. Während dieser Zeit wird der Druck allmählich auf 3 mbar reduziert. 15,2 g (0,47 Mole, 70 % der Theorie) des Reaktionsprodukts (105) werden erhalten. Der Katalysator wird durch Filtration zurückgewonnen. Man erhält ein farbloses klares Produkt.

**Beispiel C13**

Umesterung eines Glyceridgemisches mit 3,5-Bis(1,1-dimethyl)-4-hydroxyphenylpropionsäure-methylester (101).

65,0 g Kokosöl (ca. 0,096 mol), 50,9 g Glycerin 86,6% (0,48 mol), 292,4 g 3,5-bis(1,1-dimethylethyl)-4-hydroxyphenylpropionsäure-methylester (101) (1,00 mol) und 8,0 g Katalysator aus Beispiel B29 werden langsam unter Rühren in einem 1 Liter Kolben mit Dephlegmator bei 60° C, Kühler bei 15-20° C und Kühlfalle bei -80° C auf 200 ° C unter Rühren erhitzt, wobei ein Druck von 200 mbar eingestellt wird. Die Temperatur wird bei 200° C während zwei Stunden gehalten und der Druck innerhalb einer Stunde von 200 mbar auf 20 mbar reduziert. Es werden 6,6 g Wasser und 30,0 g Methanol abgezogen. Der Katalysator wird durch Filtration zurückgewonnen. 343,4 g Produkt werden als klares, gelbliches Harz erhalten.

**Patentansprüche**

1. Verfahren zur Umesterung von Carbonsäureestern, **dadurch gekennzeichnet, dass** man als Katalysator eine an einen anorganischen Träger gebundene Zinn(IV)-Verbindung, enthaltend einen Rest der Formel

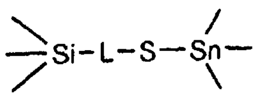

(I),

verwendet, worin L ein mindestens zweiwertiger Rest ist und mindestens eine der freien Valenzen des Si in Formel (I) an den anorganischen Täger gebunden ist.

2. Verfahren gemäss Anspruch 1 zur Herstellung von Verbindungen der Formel (X)

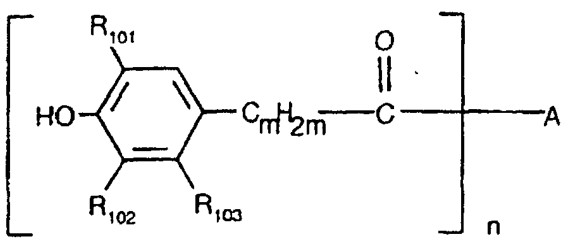

(X) ,

wobei
$R_{101}$ und $R_{102}$ gleich oder verschieden sind und H, Alkyl mit 1 bis 18 C-Atomen, Phenyl, $C_1$-$C_4$-alkylsubstituiertes Phenyl, Phenylalkyl mit 7 bis 9 C-Atomen, $C_5$-$C_{12}$-Cycloalkyl, $C_1$-$C_4$alkylsubstituiertes $C_5$-$C_{12}$-Cycloalkyl oder einen Rest

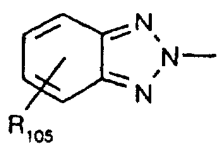

darstellen;
$R_{103}$ H oder $CH_3$ darstellt;
$R_{105}$ H, Cl, -$SO_3$H oder $C_1$-$C_4$-Alkyl darstellt;
m eine Zahl 0, 1, 2 oder 3 ist; und
n eine Zahl 1, 2, 3 oder 4 bedeutet; wobei,
wenn n = 1 ist,

A $-OR_{104}$ ist; und
$R_{104}$ die Bedeutung von Alkyl mit 2 bis 45 C-Atomen, $C_2$-$C_{45}$-Alkyl mit einem oder mehreren Sauerstoff unterbrochen, Cycloalkyl mit 5 bis 12 C-Atomen, Alkenyl mit 2 bis 18 C-Atomen, $-CH_2CHOHCH_2OC(O)R_{109}$ oder $-CH_2CH_2-OR_{106}$ hat; und
$R_{109}$ H, Alkyl mit 1 bis 8 C-Atomen, Alkenyl mit 3 bis 5 C-Atomen oder Benzyl ist; wobei
$R_{106}$

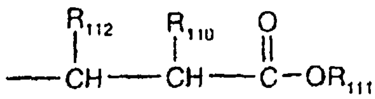 , 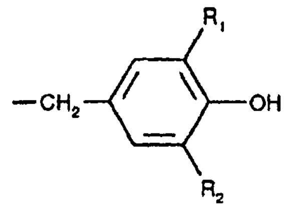

,H, Alkyl mit 1 bis 24 C-Atomen, Phenyl, Cycloalkyl mit 5 bis 12 C-Atomen oder

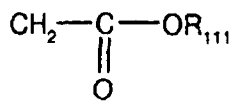

ist;
und
$R_{110}$ H oder $-CH_3$;
$R_{111}$ H oder Alkyl mit 1 bis 24 C-Atomen; und
$R_{112}$ H oder $-CH_3$ sind, mit der Massgabe, dass $R_{110}$ und $R_{112}$ nicht gleichzeitig $-CH_3$ sind; oder, wenn n = 2 ist,
A $-O-C_xH_{2x}-O-$ , $-O-(CH_2CH_2O)_aCH_2CH_2O-$ oder $-O-CH_2CH{=}CHCH_2-O-$ ist; wobei
a eine Zahl von 1 bis 30 ist; und
x eine Zahl von 2 bis 20 ist;
oder, wenn n = 3 ist,
A

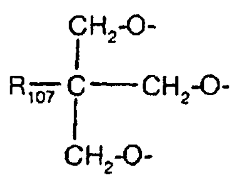

bedeutet; wobei
$R_{107}$ Alkyl mit 1 bis 24 C-Atomen oder Phenyl darstellt,
oder, wenn n = 4 ist,
A

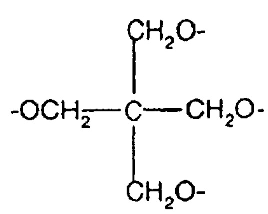

bedeutet.

3. Verfahren gemäss Anspruch 2 zur Herstellung der Verbindungen der Formel (X), worin n die Zahl eins ist;
$R_{101}$ tert. Butyl oder

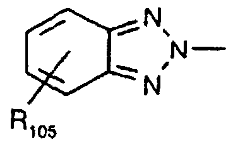

$R_{102}$ tert.-Butyl;
$R_{103}$ und $R_{105}$ H;
m die Zahl 2; A $OR_{104}$;
$R_{104}$ $(CH_2)_{17}CH_3$ ,$C_8H_{17}$ (Isomerenmischung), $C_2$-$C_{45}$-Alkyl mit einem oder mehreren Sauerstoff unterbrochen, $-CH_2CHOHCH_2OC(O)R_{109}$; und
$R_{109}$ Alkenyl mit 3 bis 5 C-Atomen ist;
oder n die Zahl 2 ist;
$R_{101}$ tert. Butyl oder

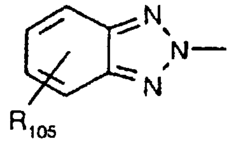

$R_{102}$ $CH_3$;
$R_{103}$ H;
m die Zahl 2;
A , $-O-(CH_2CH_2O)_aCH_2CH_2O-$ ; und
a eine Zahl von 1 bis 30 ist;
oder n die Zahl 3 ist;
$R_{101}$ und $R_{102}$ tert.-Butyl;
$R_{103}$ H;
m die Zahl 2; und
A $-O-CH_2-CH(O-)-CH_2-O-$ ist;
oder n die Zahl 4 ist;
$R_{101}$ und $R_{102}$ tert.-Butyl;
$R_{103}$ H;
m die Zahl 2; und
A $C(CH_2-O-)_4$ ist.

4. Verfahren gemäss Anspruch 1 zur Herstellung von Verbindungen der Formel (XX)

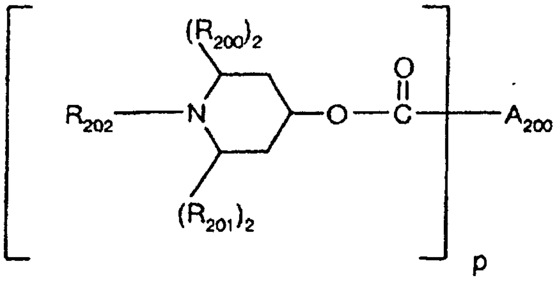
(XX) ,

worin
$R_{200}$ und $R_{201}$ unabhängig voneinander $C_1$-$C_4$-Alkyl;
$R_{202}$ H, $C_1$-$C_{18}$-Alkyl, O oder $C_1$-$C_{18}$-Alkoxy;

$A_{200}$ wenn p=1 ist, $C_1$-$C_{12}$-Alkyl, $C_1$-$C_{12}$-Alkyl substituiert mit -C(O)OH oder $C_2$-$C_5$-Alkenyl; oder, wenn p=2 ist, $C_1$-$C_{12}$-Alkylen; und p die Zahl 1 oder 2 bedeutet.

**5.** Verfahren gemäss Anspruch 1 zur Herstellung von Carbonsäureestern, durch Umsetzung einer Verbindung der Formel (XXX)

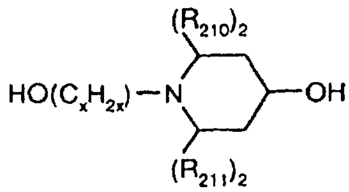

(XXX)

worin
$R_{210}$ und $R_{211}$ unabhängig voneinander $C_1$-$C_4$-Alkyl; und
x eine Zahl 1 bis 12 ist;
mit einer Verbindung der Formel

$$R_{212}OC(O)\text{-}A_{210}\text{-}(O)COR_{213} \quad \text{(XXXI),}$$

worin
$R_{212}$ und $R_{213}$ unabhängig voneinander $C_1$-$C_4$-Alkyl; und
$A_{210}$ $C_1$-$C_{12}$-Alkylen ist.

**6.** Verfahren nach Anspruch 1, wobei der Katalysator eine Verbindung oder ein Gemisch von Verbindungen ist, erhältlich durch Umsetzung von einer Verbindung der Formel (IIa)

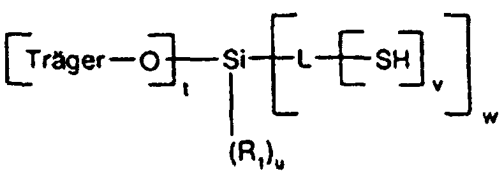

(IIa)

wobei,
t eine Zahl 1, 2, oder 3;
u eine Zahl 0, 1 oder 2;
v eine Zahl 1, 2, 3, 4, 5, 6, 7, 8 oder 9; und
w eine Zahl 1, 2 oder 3 bedeutet,
mit der Massgabe, dass t+u+w=4 ist;
L einen mindestens zweiwertigen $C_1$-$C_{18}$-Alkylen-, $C_2$-$C_{18}$-Alkenylen-, Phenylen-, $C_1$-$C_{18}$-Alkylen/Phenylen- oder durch ein oder mehrere O, C(O), S, C(S), N(Y), C(O)-N(Y), N(Y)-C(O)-N(Y) und/oder N(Y)-C(O)-O unterbrochenen $C_2$-$C_{18}$-Alkylen- oder $C_1$-$C_{18}$-Alkylen/Phenylen-Rest bedeutet;
Träger ein anorganisches Trägermaterial ist;
R, unabhängig voneinander $C_1$-$C_4$-Alkoxy, Phenoxy, $C_1$-$C_4$-Alkyl, Phenyl oder Halogen; und
Y unabhängig voneinander $C_1$-$C_{18}$-Alkyl, Phenyl- oder $C_1$-$C_{18}$-Alkyl/Phenyl bedeutet; mit einer Verbindung der Formel

$$Sn[R_2]_4 \quad \text{(IIb),}$$

wobei
$R_2$ unabhängig voneinander $C_1$-$C_{18}$-Alkyl, substituiertes $C_1$-$C_{18}$-Alkyl, $C_2$-$C_{18}$-Alkenyl, $C_1$-$C_{18}$-Halogenalkyl,

$C_6$-$C_{16}$-Halogenaryl, $C_3$-$C_{16}$-Halogenheteroaryl, $C_1$-$C_4$-Alkylphenyl, $C_1$-$C_4$-Alkoxyphenyl, Halogen-$C_1$-$C_4$-alkylphenyl, Halogen-$C_1$-$C_4$-alkoxyphenyl, $C_1$-$C_{12}$-Hydroxyalkyl, $C_3$-$C_8$-Cycloalkyl, $C_6$-$C_{16}$-Aryl, substituiertes $C_6$-$C_{16}$-Aryl, $C_7$-$C_{16}$-Aralkyl, $C_3$-$C_6$-Heterocycloalkyl, $C_3$-$C_{16}$-Heteroaryl, $C_4$-$C_{18}$-Heteroaralkyl, -Cl, -Br, -I, OH, -$OC_1$-$C_{18}$-Alkyl,-$OC_6$-$C_{16}$-Aryl, -OOC-$C_1$-$C_{18}$-Alkyl, -OOC-Phenyl, SH, -$N(Si(CH_3)_3)_2$, $SC_1$-$C_{18}$-Alkyl, -$SC_6$-$C_{16}$-Aryl, -SC(O)-$C_1$-$C_{18}$-Alkyl, -SC(O)-Phenyl, -SC(S)-$C_1$-$C_{18}$-Alkyl, -SC(S)-Phenyl, H oder eine Sauerstoffbrücke zu einem weiteren Sn Atom;
oder zwei Reste $R_2$ zusammen auch =S oder =O bedeuten,
mit der Massgabe, dass mindestens ein Rest $R_2$ vorhanden ist, welcher mit einer SH-Gruppe unter Bildung einer S-Sn Bindung reagieren kann.

**7.** Verwendung einer an einen anorganischen Träger gebundenen Zinn(IV)-Verbindung enthaltend einen Rest der Formel

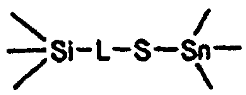

(I),

worin L ein mindestens zweiwertiger Rest ist und mindestens eine der freien Valenzen des Si in Formel (I) an den anorganischen Täger gebunden ist, als Katalysator bei der Umesterung von Carbonsäureestem.

**Claims**

**1.** Process for the transesterification of carboxylic esters, **characterized in that** the catalyst used is a tin(IV) compound comprising a radical of the formula

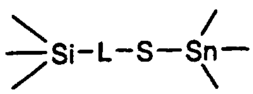

(I)

bound to an inorganic support, where L is an at least divalent radical and at least one of the free valences of the Si in formula (I) is bound to the inorganic support.

**2.** Process according to claim 1 for preparing compounds of the formula (X)

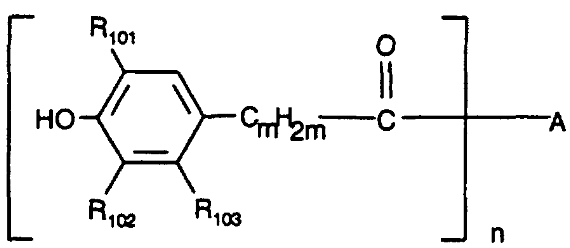

(X),

in which
$R_{101}$ and $R_{102}$ are identical or different and are H, alkyl having from 1 to 18 C atoms, phenyl, $C_1$-$C_4$alkyl-substituted phenyl, phenylalkyl having from 7 to 9 C atoms, $C_5$-$C_{12}$cycloalkyl,
$C_1$-$C_4$alkyl-substituted $C_5$-$C_{12}$cycloalkyl or a radical

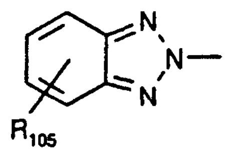 ;

$R_{103}$ is H or $CH_3$;
$R_{105}$ is H, Cl, $-SO_3H$ or $C_1$-$C_4$alkyl;
m is 0, 1, 2 or 3; and
n is 1, 2, 3 or 4; where,
when n = 1,
A is $-OR_{104}$; and
$R_{104}$ is alkyl having from 2 to 45 C atoms, $C_2$-$C_{45}$alkyl interrupted by one or more oxygen, cycloalkyl having from 5 to 12 C atoms, alkenyl having from 2 to 18 C atoms, $-CH_2CHOHCH_2OC(O)R_{109}$ or $-CH_2CH_2-OR_{106}$; and
$R_{109}$ is H, alkyl having from 1 to 8 C atoms, alkenyl having from 3 to 5 C atoms or benzyl; where
$R_{106}$ is

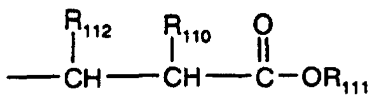 , 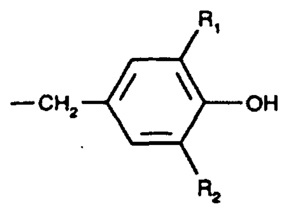 ,

H, alkyl having from 1 to 24 C atoms, phenyl, cycloalkyl having from 5 to 12 C atoms or

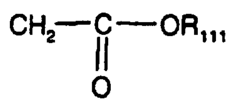 ;

and
$R_{110}$ is H or $-CH_3$;
$R_{111}$ is H or alkyl having from 1 to 24 C atoms; and
$R_{112}$ is H or $-CH_3$, with the proviso that $R_{110}$ and $R_{112}$ are not simultaneously $-CH_3$;
or, when n = 2,
A is $-O-C_xH_{2x}-O-$, $-O-(CH_2CH_2O)_aCH_2CH_2O-$ or $-O-CH_2CH=CHCH_2-O-$; in which
a is from 1 to 30; and
x is from 2 to 20;
or, when n = 3,
A is

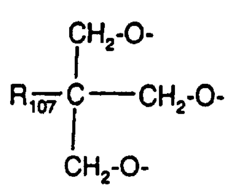 ;

in which
$R_{107}$ is alkyl having from 1 to 24 C atoms or phenyl,
or, when n = 4,

A is

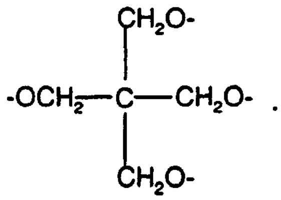

**3.** Process according to claim 2 for preparing compounds of the formula (X) in which n is one;
$R_{101}$ is tert-butyl or

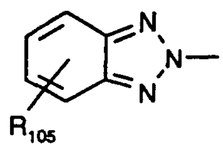

$R_{102}$ is tert-butyl;
$R_{103}$ and $R_{105}$ are H;
m is 2; A is $OR_{104}$;
$R_{104}$ is $(CH_2)_{17}CH_3$, $C_8H_{17}$ (isomer mixture), $C_2$-$C_{45}$alkyl interrupted by one or more oxygen, $-CH_2CHOHCH_2OC(O)R_{109}$; and
$R_{109}$ is alkenyl having from 3 to 5 C atoms;
or n is 2;
$R_{101}$ is tert-butyl or

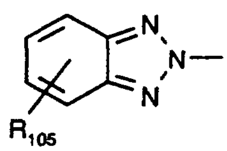

$R_{102}$ is $CH_3$;
$R_{103}$ is H;
m is 2;
A is $-O-(CH_2CH_2O)_aCH_2CH_2O-$; and
a is from 1 to 30;
or n is 3;
$R_{101}$ and $R_{102}$ are tert-butyl;
$R_{103}$ is H;
m is 2; and
A is $-O-CH_2-CH(O-)-CH_2-O-$;
or n is 4;
$R_{101}$ and $R_{102}$ are tert-butyl;
$R_{103}$ is H;
m is 2; and
A is $C(CH_2-O-)_4$.

**4.** Process according to claim 1 for preparing compounds of the formula (XX)

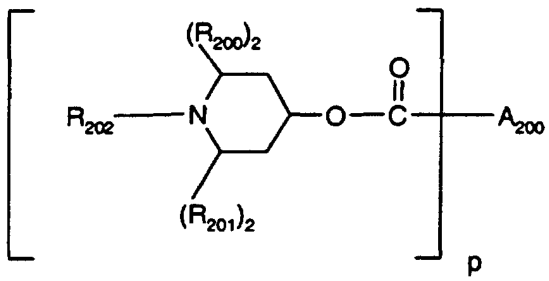

(XX),

in which
$R_{200}$ and $R_{201}$ are, independently of one another, $C_1$-$C_4$alkyl;
$R_{202}$ is H, $C_1$-$C_{18}$alkyl, O or $C_1$-$C_{18}$alkoxy;
$A_{200}$ is, when p=1, $C_1$-$C_{12}$alkyl, $C_1$-$C_{12}$alkyl substituted by -C(O)OH or $C_2$-$C_5$alkenyl; or,when p=2, $C_1$-$C_{12}$alkylene; and
p is 1 or 2.

**5.** Process according to claim 1 for preparing carboxylic esters by reaction of a compound of the formula (XXX)

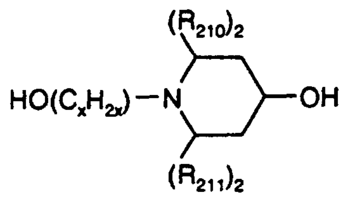

(XXX)

in which
$R_{210}$ and $R_{211}$ are, independently of one another, $C_1$-$C_4$alkyl; and
x is from 1 to 12;
with a compound of the formula

$$R_{212}OC(O)\text{-}A_{210}\text{-}(O)COR_{213} \qquad \text{(XXXI)}$$

in which
$R_{212}$ and $R_{213}$ are, independently of one another, $C_1$-$C_4$alkyl; and
$A_{210}$ is $C_1$-$C_{12}$alkylene.

**6.** Process according to claim 1, wherein the catalyst is a compound or a mixture of compounds obtainable by reaction of a compound of the formula (IIa)

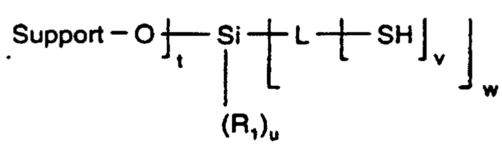

(IIa),

in which
t is 1, 2 or 3;
u is 0,1 or 2;
v is 1, 2, 3, 4, 5, 6, 7, 8 or 9; and
w is 1, 2 or 3,
with the proviso that t+u+w=4;
L is an at least divalent $C_1$-$C_{18}$alkylene, $C_2$-$C_{18}$alkenylene, phenylene,

$C_1$-$C_{18}$alkylene/phenylene radical or $C_2$-$C_{18}$alkylene or $C_1$-$C_{18}$alkylene/phenylene radical interrupted by one or more O, C(O), S, C(S), N(Y), C(O)-N(Y), N(Y)-C(O)-N(Y) and/or N(Y)-C(O)-O;
Support is an inorganic support material;
$R_1$ are, independently of one another, $C_1$-$C_4$alkoxy, phenoxy, $C_1$-$C_4$alkyl, phenyl or halogen; and
Y are, independently of one another, $C_1$-$C_{18}$alkyl, phenyl or $C_1$-$C_{18}$alkyl/phenyl; with a compound of the formula

$$Sn[R_2]_4 \qquad \text{(IIb)},$$

in which
$R_2$ are, independently of one another, $C_1$-$C_{18}$alkyl, substituted $C_1$-$C_{18}$alkyl, $C_2$-$C_{18}$alkenyl, $C_1$-$C_{18}$haloalkyl, $C_6$-$C_{16}$haloaryl, $C_3$-$C_{16}$haloheteroaryl, $C_1$-$C_4$alkylphenyl, $C_1$-$C_4$alkoxyphenyl, halo-$C_1$-$C_4$alkylphenyl, halo-$C_1$-$C_4$alkoxyphenyl, $C_1$-$C_{12}$hydroxyalkyl, $C_3$-$C_8$cycloalkyl, $C_6$-$C_{16}$aryl, substituted $C_6$-$C_{16}$aryl, $C_7$-$C_{16}$aralkyl, $C_3$-$C_6$heterocycloalkyl, $C_3$-$C_{16}$heteroaryl, $C_4$-$C_{16}$heteroaralkyl, -Cl, -Br, -I, OH, -O$C_1$-$C_{18}$alkyl, -O$C_6$-$C_{16}$aryl, -OOC-$C_1$-$C_{18}$alkyl, -OOC-phenyl, SH, -$N(Si(CH_3)_3)_2$, S$C_1$-$C_{18}$alkyl, -S$C_6$-$C_{16}$aryl, -SC(O)-$C_1$-$C_{18}$alkyl, -SC(O)-phenyl, -SC(S)-$C_1$-$C_{18}$alkyl, -SC(S)-phenyl, H or an oxygen bridge to a further Sn atom;
or two radicals $R_2$ are together =S or =O,
with the proviso that at least one $R_2$ radical which can react with an SH group to form an S-Sn bond is present.

**7.** Use of a tin(IV) compound comprising a radical of the formula

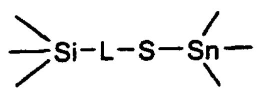

(I),

bound to an inorganic support, where L is an at least divalent radical and at least one of the free valences of the Si in formula (I) is bound to the inorganic support, as catalyst in the transesterification of carboxylic esters.

## Revendications

**1.** Procédé pour la transestérification d'esters d'acides carboxyliques, **caractérisé en ce qu'**on utilise en tant que catalyseur un composé de l'étain(IV) lié à un support inorganique, présentant un reste de formule

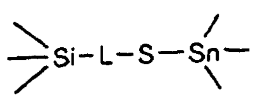

(I),

où L représente un reste au moins bivalent et au moins une valence libre de Si à la formule (I) est liée au support inorganique.

**2.** Procédé selon la revendication 1 pour la préparation de composés de formule (X)

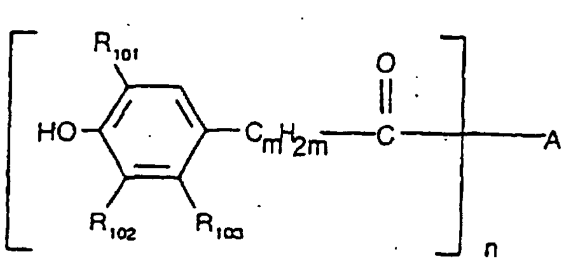

(X),

où

$R_{101}$ et $R_{102}$ sont identiques ou différents et représentent des atomes d'hydrogène, des groupes alkyle présentant 1 à 18 atomes de carbone, phényle, phényle substitué par un substituant alkyle en $C_1$-$C_4$, phénylalkyle présentant 7 à 9 atomes de carbone, cycloalkyle en $C_5$-$C_{12}$, cycloalkyle en $C_5$-$C_{12}$ substitué par un substituant alkyle en $C_1$-$C_4$ ou un reste

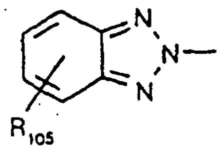

$R_{103}$ représente un atome d'hydrogène ou un groupe $CH_3$ ;
$R_{105}$ représente un atome d'hydrogène, un atome de Cl, des groupes $-SO_3H$ ou alkyle en $C_1$-$C_4$,
m vaut 0, 1, 2 ou 3 ; et
n vaut 1, 2, 3 ou 4 ; où

lorsque n = 1,

A représente un groupe $-OR_{104}$ ; et
$R_{104}$ possède la signification de groupes alkyle présentant 2 à 45 atomes de carbone, alkyle en $C_2$-$C_{45}$ interrompu par un ou plusieurs atomes d'oxygène, cycloalkyle présentant 5 à 12 atomes de carbone, alcényle présentant 2 à 18 atomes de carbone, $-CH_2CHOHCH_2OC(O)R_{109}$ ou $-CH_2CH_2-OR_{106}$ ; et
$R_{109}$ représente un atome d'hydrogène, des groupes alkyle présentant 1 à 8 atomes de carbone, alcényle présentant 3 à 5 atomes de carbone ou benzyle ; où
$R_{106}$ représente des groupes

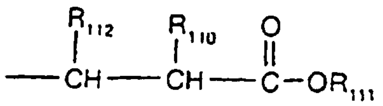 , 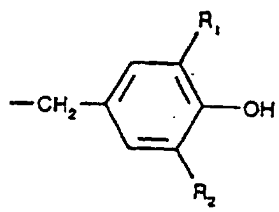

un atome d'hydrogène, des groupes alkyle présentant 1 à 24 atomes de carbone, phényle, cycloalkyle présentant 5 à 12 atomes de carbone ou

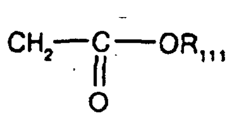 ;

et
$R_{110}$ représente un atome d'hydrogène ou un groupe $CH_3$ ;
$R_{111}$ représente un atome d'hydrogène ou un groupe alkyle présentant 1 à 24 atomes de carbone ; et
$R_{112}$ représente un atome d'hydrogène ou un groupe $CH_3$, à condition que $R_{110}$ et $R_{112}$ ne représentent pas simultanément un groupe $-CH_3$ ;

ou, lorsque n = 2,

A représente des groupes $-O-C_xH_{2x}-O-$, $-O-(CH_2CH_2O)_aCH_2CH_2O-$ou $-O-CH_2CH=CHCH_2-O-$
a va de 1 à 30 ; et
x va de 2 à 20 ;

ou, lorsque n = 3,

A représente un groupe

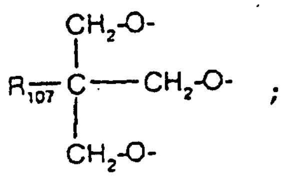

où

$R_{107}$ représente un groupe alkyle présentant 1 à 24 atomes de carbone ou phényle ;

ou, lorsque n = 4,

A représente un groupe

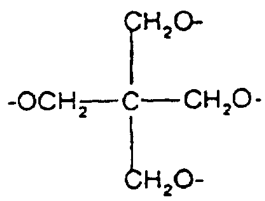

**3.** Procédé selon la revendication 2 pour la préparation des composés de formule (X), où n vaut 1 ;

$R_{101}$ représente un groupe tert-butyle ou

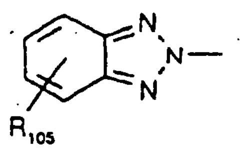

$R_{102}$ représente un groupe tert-butyle ;
$R_{103}$ et $R_{105}$ représentent un atome d'hydrogène ;
m vaut 2 ; A représente un groupe $OR_{104}$ ;
$R_{104}$ représente des groupes $(CH_2)_{17}CH_3$, $C_8C_{17}$ (mélange d'isomères), alkyle en $C_2$-$C_{45}$ interrompu par un ou plusieurs atomes d'oxygène, $-CH_2CHOHCH_2OC(O)R_{109}$ ; et
$R_{109}$ représente un groupe alcényle présentant 3 à 5 atomes de carbone ;

ou n vaut 2 ;

$R_{101}$ représente un groupe tert-butyle ou

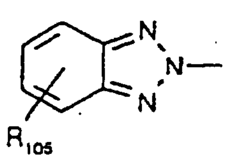

$R_{102}$ représente un groupe $CH_3$ ;
$R_{103}$ représente un atome d'hydrogène ;
m vaut 2 ;
A représente un groupe $-O-(CH_2CH_2O)_aCH_2CH_2O-$ ;

a va de 1 à 30 ;

ou n vaut 3 ;

$R_{101}$ et $R_{102}$ représentent un groupe tert-butyle ;
$R_{103}$ représente un atome d'hydrogène ;
m vaut 2 ; et
A représente un groupe $-O-CH_2CH(O-)-CH_2-O-$; et ou n vaut 4 ;
$R_{101}$ et $R_{102}$ représentent un groupe tert-butyle ;
$R_{103}$ représente un atome d'hydrogène ;
m vaut 2 ; et
A représente un groupe $-C(CH_2-O)_4$.

**4.** Procédé selon la revendication 1 pour la préparation de composés de formule (XX)

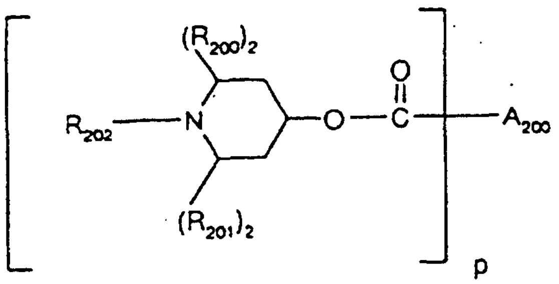

(XX) ,

où

$R_{200}$ et $R_{201}$ représentent, indépendamment l'un de l'autre, un groupe alkyle en $C_1$-$C_4$ ;
$R_{202}$ représente un atome d'hydrogène, des groupes alkyle en $C_1$-$C_{18}$, 0 ou alkoxy en $C_1$-$C_{18}$ ;
$A_{200}$ lorsque p = 1, représente des groupes alkyle en $C_1$-$C_{12}$, alkyle en $C_1$-$C_{12}$ substitué par des substituants -C(O)OH ou alcényle en $C_2$-$C_5$ ;

ou lorsque p = 2, représente un groupe alkylène en $C_1$-$C_{12}$ ; et

p vaut 1 ou 2.

**5.** Procédé selon la revendication 1 pour la préparation d'esters d'acides carboxyliques par réaction d'un composé de formule (XXX)

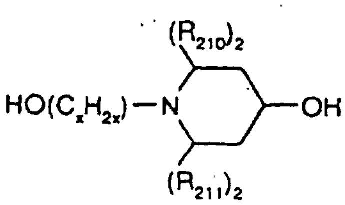

(XXX),

où

$R_{210}$ et $R_{211}$ représentent, indépendamment l'un de l'autre, un groupe alkyle en $C_1$-$C_4$ ;
x va de 1 à 12 ;

sur un composé de formule

$$R_{212}OC(O)\text{-}A_{210}\text{-}(O)COR_{213} \quad (XXXI)$$

où

$R_{212}$ et $R_{213}$ représentent, indépendamment l'un de l'autre, un groupe alkyle en $C_1$-$C_4$ ;
$A_{210}$ représente un groupe alkylène en $C_1$-$C_4$.

**6.** Procédé selon la revendication 1, où le catalyseur est un composé ou un mélange de composés, que l'on peut obtenir par réaction d'un composé de formule (IIa)

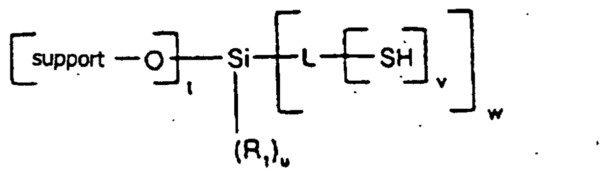

(IIa),

où

t vaut 1, 2 ou 3 ;
u vaut 0, 1 ou 2 ;
v vaut 1, 2, 3, 4, 5, 6, 7, 8 ou 9 ; et
w vaut 1, 2 ou 3,

à condition que t+u+w = 4 ;

L représente un reste au moins bivalent alkylène en $C_1$-$C_{18}$, alcénylène en $C_2$-$C_{18}$, phénylène, alkylène en $C_1$-$C_{18}$/phénylène, ou alkylène en $C_2$-$C_{18}$ ou alkylène en $C_1$-$C_{18}$/phénylène interrompu par un ou plusieurs groupes O, C(O), S, C(S), N(Y), C(O)-N(Y), N(Y)-C(O)-N(Y) et/ou N(Y)-C(O)-O ;

support signifie un support en matière inorganique ;

$R_1$ représente indépendamment des groupes alkoxy en $C_1$-$C_4$, phénoxy, alkyle en $C_1$-$C_4$, phényle ou halogène ; et
Y représente indépendamment des groupes alkyle en $C_1$-$C_{18}$, phényle ou alkyle en $C_1$-$C_{18}$/phényle ;

sur un composé de formule

$$Sn[R_2]_4 \quad (IIb)$$

$R_2$ représente indépendamment des groupes alkyle en $C_1$-$C_{18}$, alkyle en $C_1$-$C_{18}$ substitué, alcényle en $C_2$-$C_{18}$, haloalkyle en $C_1$-$C_{18}$, haloaryle en $C_6$-$C_{16}$, halohétéroaryle en $C_3$-$C_{16}$, (alkyl en $C_1$-$C_4$)-phényle, (alkoxy en $C_1$-$C_4$)-phényle, halo(alkyl en $C_1$-$C_4$)-phényle, halo(alkoxy en $C_1$-$C_4$)-phényle, hydroxyalkyle en $C_1$-$C_{12}$, cycloalkyl en $C_3$-$C_8$, aryle en $C_6$-$C_{16}$, aryle en $C_6$-$C_{16}$ substitué, aralkyle en $C_7$-$C_{16}$, hétérocycloalkyle en $C_3$-$C_6$, hétéroaryle en $C_3$-$C_{16}$, hétéroaralkyle en $C_4$-$C_{16}$, -Cl, -Br, -I, -OH, -O-alkyle en $C_1$-$C_{18}$, -O-aryle en $C_6$-$C_{16}$, -OOC-alkyle en $C_1$-$C_{18}$, -OOC-phényle, SH, $-N(Si(CH_3)_3)_2$, S-alkyle en $C_1$-$C_{18}$, -S-aryle en $C_6$-$C_{16}$, -SC(O)-alkyle en $C_1$-$C_{18}$, -SC(O)-phényle, -SC(S)-alkyle en $C_1$-$C_{18}$, -SC(S)-phényle, H ou un pont oxygène vers un autre atome de Sn ;

ou deux restes $R_2$ conjointement représentent également =S ou =O,
à condition que, au moins un reste $R_2$ qui peut réagir sur un groupe -SH en formant une liaison S-Sn, soit présent.

**7.** Utilisation d'un composé de l'étain(IV) présentant un reste de formule

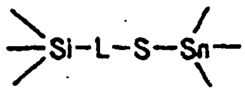

(I)

où L représente un reste au moins bivalent et au moins une des valences libres de Si à la formule (I) est liée au support inorganique,
en tant que catalyseur dans la transestérification d'esters d'acides carboxyliques.